# EUROPEAN PATENT APPLICATION

(11) **EP 4 506 328 A1**
(43) Date of publication of application: **12.02.2025**
(21) Application number: 23785015.1
(22) Date of filing: 06.04.2023
(51) Int. Cl.: C07C 49/84, C07C 49/80, C07D 311/28, C07D 307/46, C07D 333/16, C07D 215/04, C07D 307/80, C07D 407/04, C07D 409/04, A61K 31/353

(54) **COMPOSITION FOR PREVENTING OR TREATING MULTIPLE MYELOMA COMPRISING NOVEL CHROMANON COMPOUND AS ACTIVE INGREDIENT**

(30) Priority: 08.04.2022 KR 20220043870
(71) Applicant: KBlueBio Inc, Jeollanam-do 58128 (KR); Industry Foundation of Chonnam National University, Gwangju 61186 (KR)
(72) Inventor: KIM, Hye Ran, Gwangju 61698 (KR); LEE, Young Eun, Gwangju 61742 (KR); SHIN, Myung Geun, Gwangju 61143 (KR)
(74) Representative: Synergy IP Group AG
(86) International application number: PCT/KR2023/004646
(87) International publication number: WO 2023/195791

(57) **Abstract**

The present invention provides a novel compound having a chromanone or its ring-opening form, phenylpropenone, as backbone and compositions for the preventing or treating multiple myeloma, comprising the same. The compounds of the invention exhibit significant killing effects against various multiple myeloma cell lines and show *in vivo* anti-cancer effects that exceed those of lenalidomide, a commercially available immunomodulator widely used in the treatment of multiple myeloma and myelodysplastic syndromes. In addition, the compounds of the invention exhibit a significant synergistic effect when co-administered with the thalidomide or its analog lenalidomide, and thus are useful as an efficient therapeutic agent or therapeutic aid agent composition for multiple myeloma which is an incurable disease.

## Description

### Technical Field

The present invention relates to novel compounds having a chromanone or its ring-opening form, phenylpropenone, as backbone and compositions for the preventing or treating bone marrow cancer including multiple myeloma, comprising the same.

### Background Art

Multiple myeloma is a blood cancer caused by the proliferation of plasma cells, the mature form of B-lymphocytes, and is typical blood tumors along with leukemia and lymphoma. It occurs in people over the age of 65 on average, and while the incidence has been relatively low in East Asia, including Korea, it has been one of the most rapidly increasing cancers since the 2000s due to pollution, increased exposure to environmental hormones, and aging. Treatment of multiple myeloma has been based on melphalan and prednisone therapy, but the immunomodulators such as Thalidomide and Lenalidomide and the proteosome inhibitor such as Bortezomib have been widely used in clinical practice. However, these drugs destroy or inhibit the function of normal hematopoietic stem cells, causing serious side effects including pancytopenia, peripheral neuritis, emboli/thrombosis, and the development of secondary cancers. In addition, multiple myeloma has become increasingly drug-resistant and the mortality rates therefrom have increased significantly.

Accordingly, the successful treatment of multiple myeloma and the improvement of patient survival rates require the development of novel anticancer drugs with significantly reduced side effects. Therefore, the present invention is intended to provide an efficient therapeutic composition for multiple myeloma, which is an incurable blood cancer, by discovering a novel small-molecule compound that exhibits a significant killing effect on multiple myeloma cells, verifying the anticancer effect of the compound in various ways through cell line and animal experiments, and observing that the compound shows no significant toxicity to a group of peripheral blood hematopoietic stem cells derived from a normal donor.

Throughout the present specification, a number of publications and patent documents are referred to and cited. The disclosure of the cited publications and patent documents is incorporated herein by reference in its entirety to more clearly describe the state of the art to which the present invention pertains and the content of the present invention.

### DISCLOSURE

### Technical Problem

The present inventors have made intensive studies to develop an effective and side-effect-free small-molecule compound for the treatment of incurable blood cancers caused by abnormal proliferation of plasma cells, including multiple myeloma. As a result, the present inventors have found that a chromanone or phenylpropenone compounds represented by Formula (1) below exhibit selective killing effects on various multiple myeloma cell lines and have significant therapeutic effects that exceed those of commercially available drugs *in vitro,* thereby completing the present invention.

Therefore, an object of the present invention is to provide a novel chromanone or phenylpropenone compound represented by Formula (1) below and a composition for preventing or treating multiple myeloma comprising the same as an active ingredient.

Other objects and advantages of the present invention will be more apparent from the following detailed description, the appended claims, and the accompanying drawings.

### Technical Solution

In one aspect of this invention, there is provided a compound represented by the following Formula 1 or a pharmaceutically acceptable salt thereof:

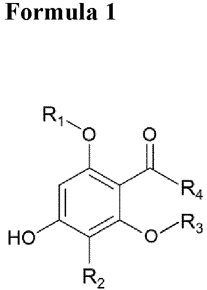

wherein R₁ is C₁-C₃ alkyl; R₂ is hydrogen or 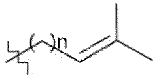 (where n is an integer of 0 to 2); R₃ is hydrogen and R₄ is 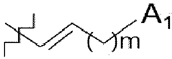 (where m is an integer of 0 to 2 and A₁ is C₆-C₁₀ aryl or 5 to 10-membered heteroaryl unsubstituted or substituted with hydroxy, halogen, C₁-C₃ alkyl, nitro, or 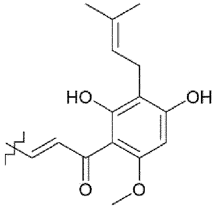 ), or R₃ and R₄ are bonded to each other to form a 6-membered heterocycloalkyl or heterocycloalkene substituted with C₆-C₁₀ aryl or 5 to 10-membered heteroaryl unsubstituted or substituted with hydroxy, halogen or C₁ -C₃ alkyl.

The present inventors have made intensive studies to develop an effective and side-effect-free small-molecule compound for the treatment of incurable blood cancers caused by abnormal proliferation of plasma cells, including multiple myeloma. As a result, the present inventors have found that a chromanone or phenylpropenone compounds represented by Formula (1) above exhibit selective killing effects on various multiple myeloma cell lines and have significant therapeutic effects that exceed those of commercially available drugs *in vivo.*

As used herein, the term "alkyl" refers to a straight-chain or branched saturated hydrocarbon group, and includes, for example, methyl, ethyl, propyl, isopropyl, etc. The term "C₁-C₃ alkyl" refers to an alkyl group having an alkyl unit having 1 to 3 carbon atoms, and when the C₁-C₃ alkyl is substituted, the carbon atom number of the substituent is not included.

As used herein, the term "halogen" refers to an element of the halogen group and includes, for example, fluoro, chloro, bromine, and iodine. Specifically, the halogen of the present invention is bromine.

As used herein, the term "heteroaryl" refers to a heterocyclic aromatic group that contains oxygen, sulfur or nitrogen as a heteroatom in the ring. The number of heteroatoms is 1-3, more specifically 1-2. The term "5 to 10-membered heteroaryl" refers to a heteroaryl having 5 to 10 atoms forming the ring, including both carbon and hetero atoms, which may be a monocycle or a bicycle.

According to a specific embodiment, the heteroaryl is selected from the group consisting of thiophenes, furans, benzofurans and quinolines.

According to a specific embodiment, n is 1 and m is 0.

According to a specific embodiment, the heterocycloalkyl is oxanone, and the heterocycloalkene is dihydropyranone.

According to a specific embodiment, the compound represented by Formula 1 above is the compound represented by the following Formula 2: 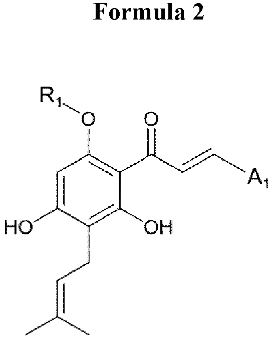 wherein R₁ and A₁ are as defined in Formula 1.

According to a specific embodiment, the compound represented by Formula 1 above is the compound represented by the following Formula 3: 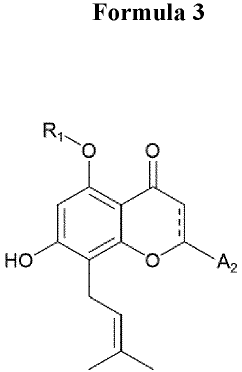 wherein R₁ is as defined in Formula 1, - - - is a single- or double bond, A₂ is C₆-C₁₀ aryl or 5 to 10-membered heteroaryl unsubstituted or substituted with hydroxy, halogen or C₁ -C₃ alkyl.

More specifically, the compound represented by Formula 1 is selected from the group consisting of compounds represented by the following Formula 4 to 33:

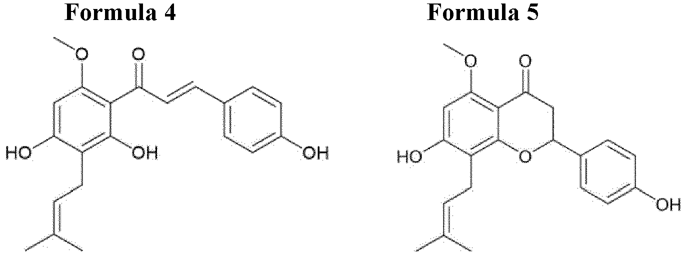

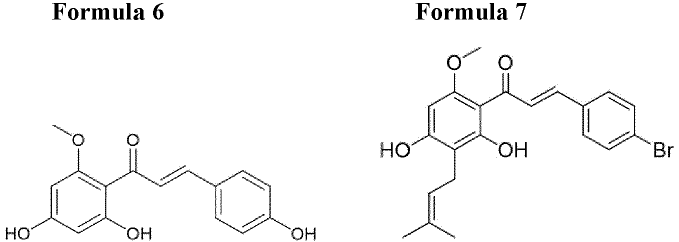

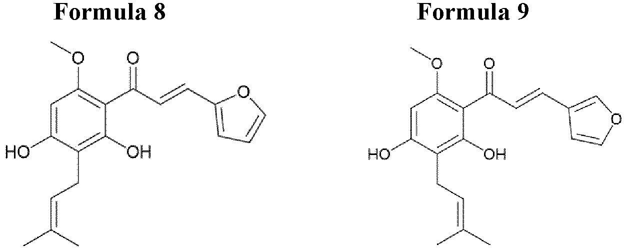

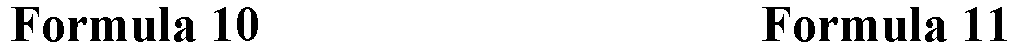

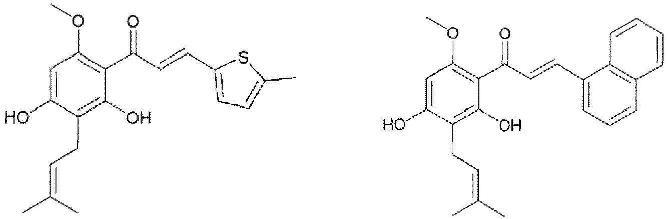

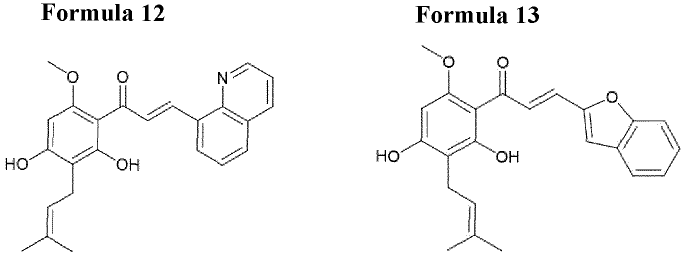

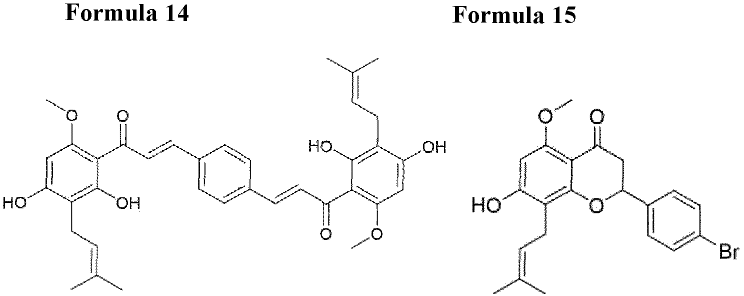

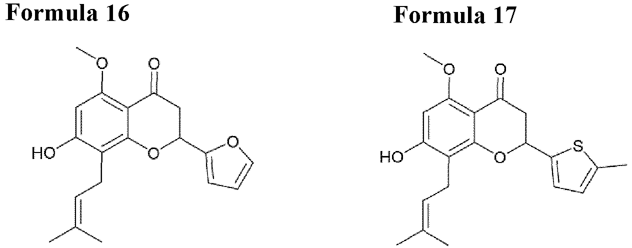

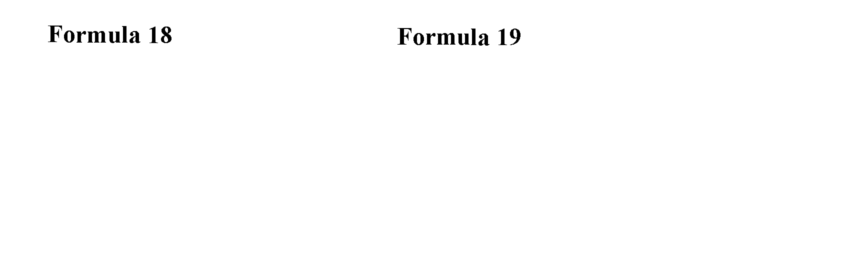

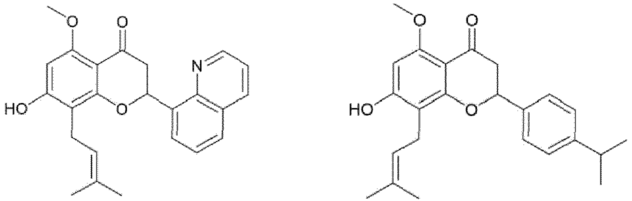

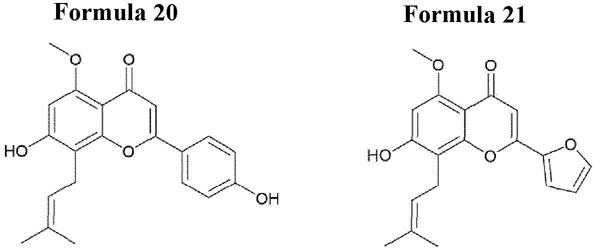

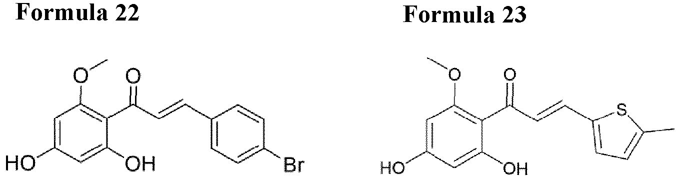

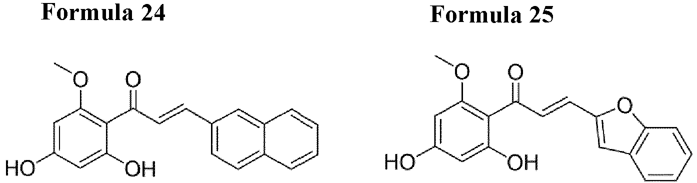

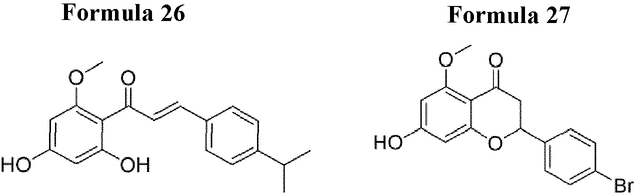

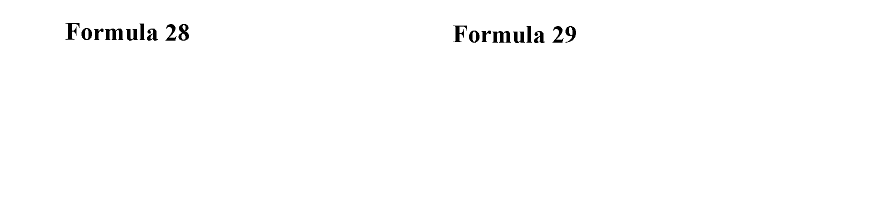

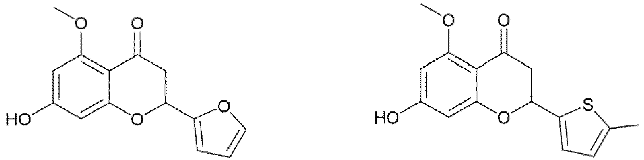

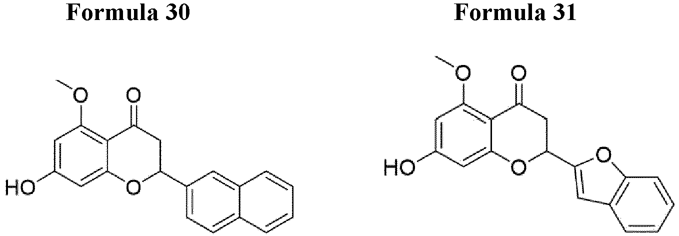

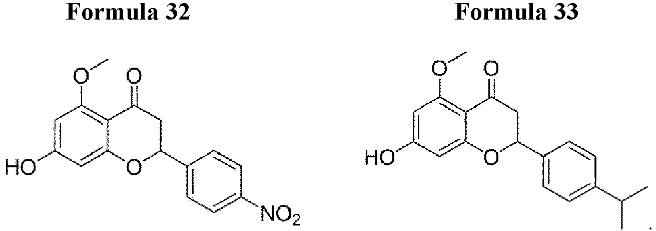

In the present specification, the term "pharmaceutically acceptable salt" includes a salt derived from a pharmaceutically acceptable inorganic acid, organic acid, or base. Examples of suitable acids include hydrochloric acid, hydrobromic acid, sulfuric acid, nitric acid, perchloric acid, fumaric acid, maleic acid, phosphoric acid, glycolic acid, lactic acid, salicylic acid, succinic acid, toluene-p-sulfonic acid, tartaric acid, acetic acid, trifluroacetic acid, citric acid, methanesulfonic acid, formic acid, benzoic acid, malonic acid, naphthalene-2-sulfonic acid, benzenesulfonic acid, and the like. Salts derived from suitable bases include salts of alkali metals such as sodium, alkaline earth metals such as magnesium, ammonium, and the like.

In another aspect of this invention, there is provided a composition for preventing or treating cancer comprising the compound of the invention described above or a pharmaceutically acceptable salt thereof, as an active ingredient.

In still another aspect of this invention, there is provided a method for preventing or treating cancer comprising administering to a subject in need thereof a composition comprising the compound of the invention described above or a pharmaceutically acceptable salt thereof, as an active ingredient.

As used herein, the term "prevention" refers to inhibiting the occurrence of a disorder or a disease in a subject who has never been diagnosed as having the disorder or disease, but is likely to suffer from such disorder or disease.

As used herein, the term "treatment" refers to (a) inhibiting the progress of a disorder, disease or symptom; (b) alleviating the disorder, disease or symptom; or (c) eliminating the disorder, disease or symptom. The composition of the present invention functions to inhibit the proliferation of tumor cell lines, induce apoptosis, and reduce the tumor lesion area, thereby inhibiting the progress of symptoms caused by tumors, or eliminating or alleviating the symptoms. Thus, the composition of the present invention may serve as a therapeutic composition for the disease alone, or may be administered in combination with other pharmacological ingredients having anticancer effects and applied as a therapeutic aid for the disease. Accordingly, as used in the present specification, the term "treatment" or "therapeutic agent" encompasses the meaning of "treatment aid" or "therapeutic aid agent".

As used herein, the term "administer" refers to administration of a therapeutically effective amount of the composition of the present invention directly to a subject so that the same amount is formed in the subject's body.

As used herein, the term "therapeutically effective amount" refers to an amount of the composition sufficient to provide a therapeutic or prophylactic effect to a subject to whom/which the composition of the present invention is to be administered. Accordingly, the term "therapeutically effective amount" is meant to include a "prophylactically effective amount".

As used herein, the term "subject" includes, without limitation, humans, mice, rats, guinea pigs, dogs, cats, horses, cows, pigs, monkeys, chimpanzees, baboons or rhesus monkeys. Specifically, the subject of the present invention is a human.

According to a specific embodiment, the cancer to be prevented or treated by the composition of the present invention is blood cancer.

As used herein, the term "blood cancer" refers to a malignant tumor that occurs in white blood cells, red blood cells, and platelets, which are components of blood; bone marrow where blood is produced; or the lymphatic system including lymphocytes, lymph nodes, and lymphatic vessels, which make up the immune system.

More specifically, the blood cancer to be prevented or treated by the composition of the present invention is bone marrow cancer, and most specifically, multiple myeloma.

As used herein, the term "multiple myeloma" refers to blood cancer that is caused by abnormal differentiation and proliferation of plasma cells, which is a type of white blood cell responsible for the immune system, in the bone marrow.

According to a specific embodiment, the composition of the present invention further comprises a compound represented by the following Formula 34 or a pharmaceutically acceptable salt thereof:

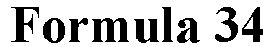

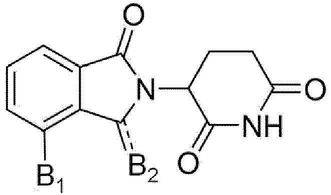

Wherein B₁ is hydrogen or -NH₂, - - - is a single bond or double bond, and B₂ is hydrogen when - - - is a single bond, or B₂ is oxygen when - - - is a double bond.

More specifically, B₁ is -NH₂, and B₂ is hydrogen. According to the octet rule, it is obvious that when B₂ is hydrogen, - - - represents a single bond. The compound of Formula 3 where B₁ is -NH₂ and B₂ is hydrogen, is lenalidomide (C₁₃H₁₃N₃O₃; 3-(4-amino-1-oxoisoindolin-2-yl)piperidine-2,6-dione).

Lenalidomide is an immunomodulator widely used in the treatment of multiple myeloma and myelodysplastic syndrome, and has anti-angiogenic and anti-inflammatory activities. According to the present invention, the compound of the present invention not only exhibited a therapeutic effect equal to or higher than that of lenalidomide, a representative commercially available therapeutic agent for multiple myeloma, but also exhibited a significant synergistic effect when co-administered with lenalidomide. Accordingly, the compound of the present invention may not only be applied as an anticancer agent alone, but may also be useful as an efficient therapeutic aid agent that is co-administered with lenalidomide or similar immunomodulators.

According to another specific embodiment, B₁ is hydrogen and B₂ is oxygen. According to the octet rule, it is obvious that when B₂ is oxygen, - - - represents a double bond. The compound of Formula 3, wherein B₁ is hydrogen and B₂ is oxygen, is thalidomide (C₁₃H₁₀N₂O₄; 2-(2,6-dioxopiperidin-3-yl)-1H-isoindole-1,3(2H)-dione).

Thalidomide is an analogue, from which lenalidomide is derived, and has been used for a long time in the treatment of multiple myeloma until lenalidomide was approved in the United States in 2005.

When the composition of the present invention is prepared as a pharmaceutical composition, the pharmaceutical composition of the present invention comprises a pharmaceutically acceptable carrier. Examples of the pharmaceutically acceptable carrier that is comprised in the pharmaceutical composition of the present invention include, but are not limited to, lactose, dextrose, sucrose, sorbitol, mannitol, starch, gum acacia, calcium phosphate, alginate, gelatin, calcium silicate, microcrystalline cellulose, polyvinylpyrrolidone, cellulose, water, syrup, methyl cellulose, methylhydroxybenzoate, propylhydroxybenzoate, talc, magnesium stearate, and mineral oil, which are commonly used in formulation. The pharmaceutical composition of the present invention may further comprise a lubricant, a wetting agent, a sweetening agent, a flavoring agent, an emulsifying agent, a suspending agent, a preservative, and the like, in addition to the above-described ingredients. Suitable pharmaceutically acceptable carriers and agents are described in detail in Remington's Pharmaceutical Sciences (19^{th} ed., 1995).

The pharmaceutical composition of the present invention may be administered via various routes. Specifically, it may be administered parenterally. More specifically, it may be administered orally, intravenously, intra-arterially, subcutaneously, intraperitoneally, intradermally, intramuscularly, intracerebroventricularly, intrathecally, by inhalation, intranasally, intra-articularly, or topically.

An appropriate dosage of the pharmaceutical composition of the present invention may vary depending on various factors such as formulation method, administration mode, patient's age, weight, sex, pathological condition, diet, administration time, administration route, excretion rate, and reaction sensitivity. A preferred dosage of the pharmaceutical composition of the present invention is within the range of 0.0001 to 100 mg/kg for an adult.

The pharmaceutical composition of the present invention may be prepared in a unit dose form or prepared to be contained in a multi-dose container by formulating with a pharmaceutically acceptable carrier and/or excipient, according to a method that may be easily carried out by a person skilled in the art. In this case, the formulation of the pharmaceutical composition may be a solution, suspension, syrup or emulsion in oil or aqueous medium, or an extract, powder, granule, tablet or capsule, and may further comprise a dispersing agent or a stabilizer.

In still another aspect of this invention, there is provided an anticancer composition for co-administration with a compound represented by the following Formula 34 or a pharmaceutically acceptable salt thereof, comprising the compound of the invention described above or a pharmaceutically acceptable salt thereof, as an active ingredient:

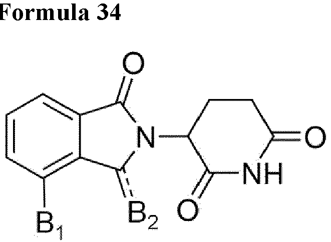

In still another aspect of this invention, there is provided a method for preventing or treating cancer comprising co-administering to a subject in need thereof the compound of the invention described above or a pharmaceutically acceptable salt thereof, and the compound represented by Formula 34 or a pharmaceutically acceptable salt thereof.

The compounds of the invention described above and the compounds of Formula 34 used in the present invention and the type of cancer to be prevented or treated by these compounds have already been described in detail above, and are hereby omitted to avoid undue redundancy.

As described above, the compound of the present invention exhibited a significant synergistic effect when co-administered with lenalidomide. Thus, the compound of the present invention may be co-administered with phthalimide-based compounds, including lenalidomide, to maximize the anticancer effect and improve patient survival rates.

Co-administration may be performed by administering a single formulation comprising both the compound of Formula 1 and compound of Formula 34 according to the present invention, or may be performed by administering separate formulations comprising each individual compound simultaneously or sequentially in any order with an appropriate time gap.

### Advantageous Effects

The features and advantages of the present invention are summarized as follows:
(a) The present invention provides a novel compound having a chromanone or its ring-opening form, phenylpropenone, as backbone and compositions for the preventing or treating multiple myeloma, comprising the same.
(b) The compounds of the invention exhibit significant killing effects against various multiple myeloma cell lines and show *in vivo* anti-cancer effects that exceed those of lenalidomide, a commercially available immunomodulator widely used in the treatment of multiple myeloma and myelodysplastic syndromes.
(c) In addition, the compounds of the invention exhibit a significant synergistic effect when co-administered with the thalidomide or its analog lenalidomide, and thus are useful as an efficient therapeutic agent or therapeutic aid agent composition for multiple myeloma which is an incurable disease.

### Brief Description of Drawings

FIG. 1 shows the change in cell viability of IM9 (FIG. 1a), RPMI 8226 (FIG. 1b), ARH 77 (FIG. 1c) and U266 (FIG. 1d) cell lines upon treatment with different concentrations of KBB-NX compounds.
FIG. 2 represents the respective cell death (apoptosis) rates of RPMI 8226 (FIG. 2a), IM9 (FIG. 2b) ARH 77 (FIG. 2c), and U266 (FIG. 2d) cell lines upon treatment with different concentrations of KBB-NX compounds.
FIG. 3 shows the change in cell viability of IM9 (FIG. 3a), RPMI 8226 (FIG. 3b), ARH 77 (FIG. 3c) and U266 (FIG. 3d) cell lines upon treatment with different concentrations of KBB-NX-1 compound.
FIG. 4 shows the change in cell viability of RPMI 8226 (FIG. 4a), IM9 (FIG. 4b), ARH 77 (FIG. 4c), and U266 (FIG. 4d) cell lines upon treatment with different concentrations of KBB-NX-2 compound.
FIG. 5 represents the respective cell death rates of RPMI 8226 (FIG. 5a), IM9 (FIG. 5b), ARH 77 (FIG. 5c), and U266 (FIG. 5d) cell lines upon treatment with different concentrations of KBB-NX-1 and KBB-NX-2 compounds.
FIG. 6 shows the change in cell viability of RPMI 8226 (FIG. 6a), IM9 (FIG. 6b), ARH 77 (FIG. 6c) and U266 (FIG. 6d) cell lines upon treatment with different concentrations of KBB-NX-4, KBB-NX-4, and KBB-NX-5 compounds, respectively.
FIG. 7 represents the respective cell death rates of RPMI 8226 (FIG. 7a), IM9 (FIG. 7b), and U266 (FIG. 7c) cell lines upon treatment with different concentrations of KBB-NX-4, KBB-NX-4 and KBB-NX-5 compounds.
FIG. 8 shows the change in cell viability of RPMI 8226 (FIG. 8a), IM9 (FIG. 8b), and U266 (FIG. 8c) cell lines upon treatment with different concentrations of KBB-NX-6, KBB-NX-7 and KBB-NX-8 compounds.
FIG. 9 represents the respective cell death rates of RPMI 8226 (FIG. 9a), IM9 (FIG. 9b), and U266 (FIG. 9c) cell lines upon treatment with different concentrations of KBB-NX-6, KBB-NX-7 and KBB-NX-8 compounds.
FIG. 10 shows the change in cell viability of each of the RPMI 8226IM9 (FIG. 10a), IM9 (FIG. 10b), and U266 (FIG. 10c) cell lines upon treatment with different concentrations of KBB-NX-9, KBB-NX-10, KBB-NX-11 and KBB-NX-12 compounds.
FIG. 11 shows the respective cell death rates of RPMI 8226 (FIG. 11a), IM9 (FIG. 11b) and U266 (FIG. 11c) cell lines following treatment with different concentrations of KBB-NX-9, KBB-NX-10, KBB-NX-11, and KBB-NX-12 compounds.
FIG. 12 shows the change in cell viability of RPMI 8226 (FIG. 12a), IM9 (FIG. 12b), and U266 (FIG. 12c) cell lines upon treatment with different concentrations of KBB-NX-13 compound.
Figure 13 represents the respective cell death rates of RPMI 8226 (FIG. 13a), IM9 (FIG. 13b), and U266 (FIG. 13c) cell lines upon treatment with different concentrations of KBB-NX-13 compound.
FIG. 14 shows the change in cell viability of RPMI 8226 (FIG. 14a), IM9 (FIG. 14b), and U266 (FIG. 14c) cell lines upon treatment with different concentrations of KBB-NX-14, KBB-NX-15, and KBB-NX-16 compounds.
Figure 15 represents the respective cell death rates of RPMI 8226 (FIG. 15a), IM9 (FIG. 15b), and U266 (FIG. 15c) cell lines upon treatment with different concentrations of KBB-NX-14, KBB-NX-15, and KBB-NX-16 compounds.
FIG. 16 shows the change in cell viability of IM9 (FIG. 16a) and U266 (FIG. 16b) cell lines upon treatment with different concentrations of KBB-NX-17 and KBB-NX-18 compounds.
FIG. 17 represents the respective cell death rates of IM9 (FIG. 17a) and U266 (FIG. 17b) cell lines upon treatment with different concentrations of KBB-NX-17 and KBB-NX-18 compounds.
FIG. 18 shows the change in cell viability of IM9 (FIG. 18a) and U266 (FIG. 18b) cell lines upon treatment with different concentrations of KBB-NX-19 and KBB-NX-20 compounds.
FIG. 19 represents the respective cell death rates of IM9 (FIG. 19a) and U266 (FIG. 19b) cell lines upon treatment with different concentrations of KBB-NX-19 and KBB-NX-20 compounds.
FIG. 20 shows the change in cell viability of IM9 (FIG. 20a) and U266 (FIG. 20b) cell lines upon treatment with different concentrations of KBB-NX-21 and KBB-NX-22 compounds, respectively.
FIG. 21 represents the respective cell death rates of IM9 (FIG. 21a) and U266 (FIG. 21b) cell lines upon treatment with different concentrations of KBB-NX-21 and KBB-NX-22 compounds.
FIG. 22 shows the change in cell viability of IM9 (FIG. 22a) and U266 (FIG. 22b) cell lines upon treatment with different concentrations of KBB-NX-23 and KBB-NX-24 compounds, respectively.
FIG. 23 represents the respective cell death rates of IM9 (FIG. 23a) and U266 (FIG. 23b) cell lines upon treatment with different concentrations of KBB-NX-23 and KBB-NX-24 compounds.
FIG. 24 shows the change in cell viability of IM9 (FIG. 24a) and U266 (FIG. 24b) cell lines upon treatment with different concentrations of KBB-NX-25, KBB-NX-26, and KBB-NX-27 compounds.
FIG. 25 shows the change in cell viability of IM9 (FIG. 25a) and U266 (FIG. 25b) cell lines upon treatment with different concentrations of KBB-NX-28 and KBB-NX-29 compounds, respectively.
FIG. 26 shows the change in cell viability of IM9 (FIG. 26a) and U266 (FIG. 26b) cell lines upon treatment with different concentrations of KBB-NX-30 and KBB-NX-31 compounds, respectively.
FIG. 27 represents the effect of treatment of multiple myeloma cells with KBB-NX compounds on killing multiple myeloma cells by inactivating PHB function, increasing intracellular reactive oxygen species production, promoting apoptosis, and arresting the cell cycle at the subG1 stage.
FIG. 28 is a schematic diagram of a test procedure for evaluating the efficacy of a compound of the invention using a mouse model of multiple myeloma.
FIG. 29 is an illustration of *in vivo* multiple myeloma cell killing by a compound of the present invention using a multiple myeloma mouse model.
Figure 30 shows the results for measurement of ALT (FIG. 30a), a marker of hepatotoxicity, and BUN (FIG. 30b), markers of renal toxicity, respectively. These markers are considered as meaningful in the initial acute toxicity evaluation for KBB-NX-16 and KBB-NX-20 compounds.

### Mode for Invention

Hereinafter, the present invention will be described in more detail by way of examples. These examples are only for explaining the present invention in more detail, and it will be apparent to those skilled in the art that the scope of the present invention according to the subject matter of the present invention is not limited by these examples.

### Examples

### Synthesis of Compounds

**[Table 1]**

| Synthetic Prenylflavonoid Class Small Molecules | | |
|---|---|---|
| **Entry** | **Structure** | **IUPAC Nomenclature** |
| KBB- NX | 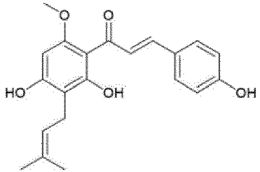 | (E)-1-(2,4-dihydroxy-6-methoxy-3-(3-methylbut-2-en-1-yl)phenyl)-3-(4-hydroxyphenyl)prop-2-en-1-one |
| KBB-NX-1 | 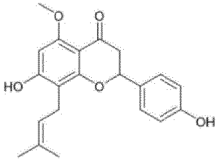 | 7-hydroxy-2-(4-hydroxyphenyl)-5-methoxy-8-(3 -methylbut-2-en-1-yl)chroman-4-one |
| KBB-NX-2 | 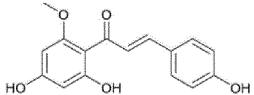 | (E)-1-(2,4-dihydroxy-6-methoxyphenyl)-3-(4-hydroxyphenyl)prop-2-en-1-one |
| KBB-NX-26 | 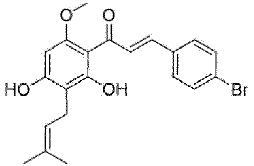 | (E)-3-(4-bromophenyl)-1-(2,4-dihydroxy-6-methoxy-3-(3-methylbut-2-en-1-yl)phenyl)prop-2-en-1-one |
| KBB-NX-9 | 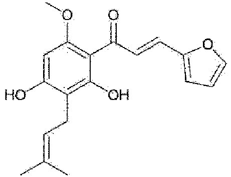 | (E)-1-(2,4-dihydroxy-6-methoxy-3-(3-methylbut-2-en-1-yl)phenyl)-3-(furan-2-yl)prop-2-en-1-one |
| KBB-NX-13 | 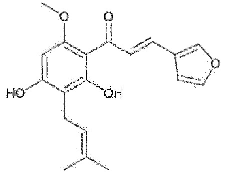 | (E)-1-(2,4-dihydroxy-6-methoxy-3-(3-methylbut-2-en-1-yl)phenyl)-3-(furan-3-yl)prop-2-en-1-one |
| KBB-NX-11 | 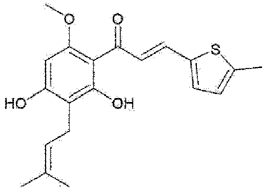 | (E)-1-(2,4-dihydroxy-6-methoxy-3-(3-methylbut-2-en-1-yl)phenyl)-3-(5-methylthiophene-2-yl)prop-2-en-1-one |
| KBB-NX-7 | 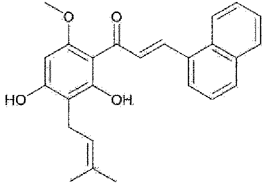 | (E)-1-(2,4-dihydroxy-6-methoxy-3-(3-methylbut-2-en-1-yl)phenyl)-3-(naphthalene-1-yl)prop-2-en-1-one |
| KBB-NX-14 | 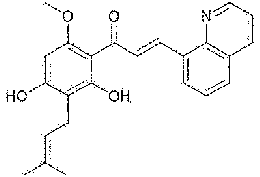 | (E)-1-(2,4-dihydroxy-6-methoxy-3-(3-methylbut-2-en-1-yl)phenyl)-3-(quinolin-8-yl)prop-2-en-1-one |
| KBB-NX-8 | 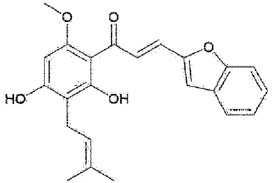 | (E)-3-(benzofuran-2-yl)-1-(2,4-dihydroxy-6-methoxy-3-(3-methylbut-2-en-1-yl)phenyl)prop-2-en-1-one |
| KBB-NX-15 | 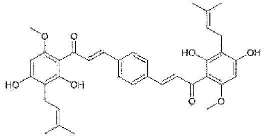 | (2E,2'E)-3,3'-(1,4-phenylene)bis(1-(2,4-dihydroxy-6-methoxy-3-(3-methylbut-2-en-1-yl)phenyl)prop-2-en-1-one) |
| KBB-NX-27 | 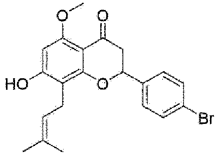 | 2-(4-bromophenyl)-7-hydroxy-5-methoxy-8-(3-methylbut-2-en-1-yl)chroman-4-one |
| KBB-NX-3 | 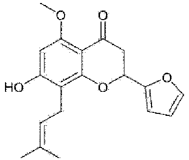 | 2-(furan-2-yl)-7-hydroxy-5-methoxy-8-(3-methylbut-2-en-1-yl)chroman-4-one |
| KBB-NX-4 | 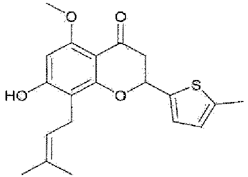 | 7-hydroxy-5-methoxy-8-(3-methylbut-2-en-1-yl)-2-(5-methylthiophene-2-yl)chroman-4-one |
| KBB- NX-5 | 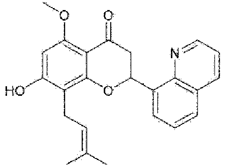 | 7-hydroxy-5-methoxy-8-(3-methylbut-2- en-1-yl)-2-(quinolin-8-yl)chroman-4-one |
| KBB- NX-16 | 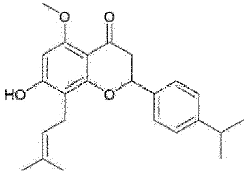 | 7-hydroxy-2-(4-isopropylphenyl)-5- methoxy-8-(3 -methylbut-2-en-1- yl)chroman-4-one |
| KBB- NX-6 | 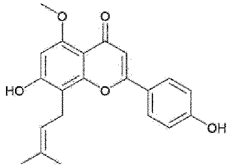 | 7-hydroxy-2-(4-hydroxyphenyl)-5- methoxy-8-(3 -methylbut-2-en-1 -yl)-4H-v chromen-4-one |
| KBB- NX-10 | 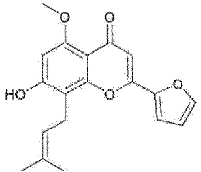 | 2-(furan-2-yl)-7-hydroxy-5-methoxy-8-(3- methylbut-2-en-1-yl)-4H-chromen-4-one |
| KBB- NX-28 | 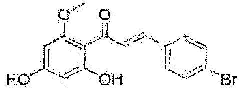 | (E)-3-(4-bromophenyl)-1-(2,4-dihydroxy- 6-methoxyphenyl)prop-2-en-1-one |
| KBB- NX-19 | 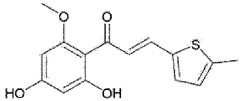 | (E)-1-(2,4-dihydroxy-6-methoxyphenyl)-3- (5-methylthiophene-2-yl)prop-2-en-1-one |
| KBB- NX-21 | 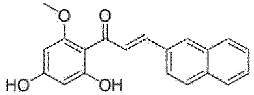 | (E)-1-(2,4-dihydroxy-6-methoxyphenyl)-3- (naphthalene-2-yl)prop-2-en-1-one |
| KBB- NX-23 | 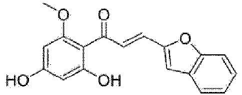 | (E)-3-(benzofuran-2-yl)-1-(2,4-dihydroxy- 6-methoxyphenyl)prop-2-en-1-one |
| KBB- NX-30 | 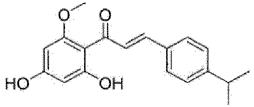 | (E)-1-(2,4-dihydroxy-6-methoxyphenyl)-3- (4- 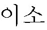 propylphenyl)prop-2-en-1-one |
| KBB- NX-28 | 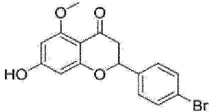 | 2-(4-bromophenyl)-7-hydroxy-5- methoxychroman-4-one |
| KBB-NX-18 | 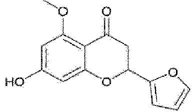 | 2-(furan-2-yl)-7-hydroxy-5-methoxychroman-4-one |
| KBB-NX-20 | 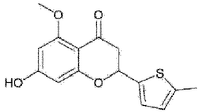 | 7-hydroxy-5-methoxy-2-(5-methylthiophene-2-yl)chroman-4-one |
| KBB-NX-22 | 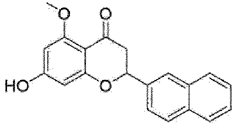 | 7-hydroxy-5-methoxy-2-(naphthalene-2-yl)chroman-4-one |
| KBB-NX-24 | 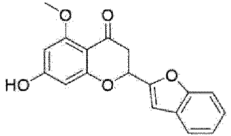 | 2-(benzofuran-2-yl)-7-hydroxy-5-methoxychroman-4-one |
| KBB-NX-25 | 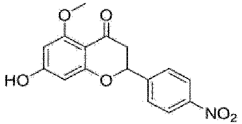 | 7-hydroxy-5-methoxy-2-(4-nitrophenyl)chroman-4-one |
| KBB-NX-31 | 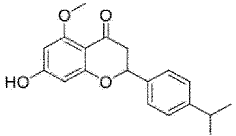 | 7-hydroxy-2-(4-isopropylphenyl)-5-methoxychroman-4-one |

### Overall Synthesis scheme 1

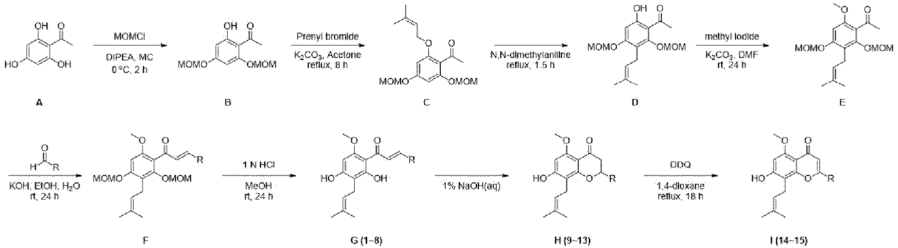

### Overall Synthesis scheme 2

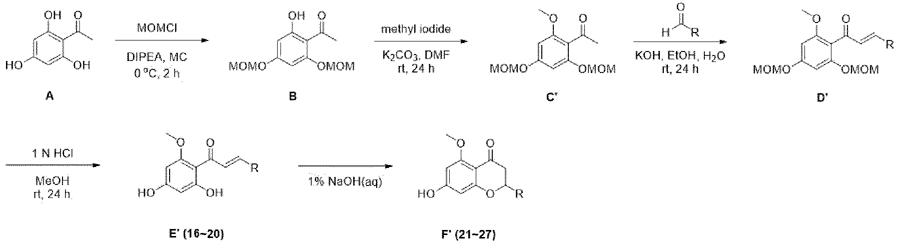

### Synthesis Example 1

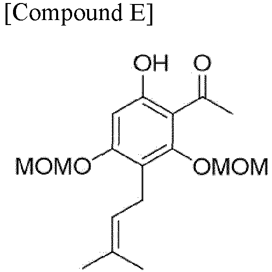

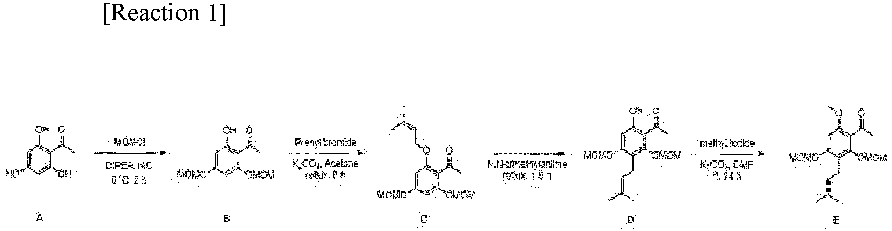

### (i) Synthesis of Compound B

25.0 g (0.135 mol) of 2,4,6-trihydroxyacetophenone (Compound A) and 87.129 g (0.674 mol) of N,N-diisopropylethylamine (DIPEA) were dissolved in 1100 ml of MC. After the solution was cooled to 0 °C, 43.418 g (0.539 mol) of chloromethylmethylether (MOMCl) was slowly added and stirred at room temperature for 2 hr. Obtained product was purified separately by silica gel column chromatography (EA:Hex = 1:4 volume ratio) to give 29.6 g of 1-(2-hydroxy-4,6-bis(methoxymethoxy)phenyl)ethan-1-one (compound B, yield 85.7%).

### (ii) Synthesis of Compound C

29.6 g (0.116 mol) of the obtained compound B was dissolved in 1200 ml of acetone, to which 28.69 g (0.193 mol) of phenyl bromide and K₂ CO₃ 63.857 g (0.462 mol) was added. The inside of the flask was quenched with Ar and heated to reflux for 8 hours. Obtained product was purified by silica gel column chromatography (EA:Hex = 1:4 volume ratio) to give 36.4 g of 1-(2,4-bis(methoxymethoxy)-6-((3-methylbut-2-en-1-yl)oxy)phenyl)ethan-1-one (compound C) in 97.1% yield.

### (iii) Synthesis of Compound D

To 36.4 g (0.112 mol) of the obtained compound C was added 205.4 g (1.694 mol) of N, N-diethylaniline for Ar-substitution inside the flask. The mixture was stirred at 220 °C for 1.5 hours, and then extracted with 10% aqueous HCl and EA and moisture was removed with MgSO₄. The obtained product was purified separately by silica gel column chromatography (EA:Hex = 1:4 volume ratio) to give 1-(6-hydroxy-2,4-bis (methoxymethoxy)-3-(3-methylbut-2-en-1-yl)phenyl)ethan-1-one (compound D) 25.2 g (yield 69.1%).

### (iv) Synthesis of Compound E

To 25.2 g (77.689 mmol) of the obtained compound D in 240 ml of DMF was added 27.6 g (0.194 mol) of methyl iodide and 21.474g (0.155 mol) of K₂CO₃ and the inside of the flask was quenched with Ar. The reaction mixture was then stirred at room temperature for 24 hours. The obtained product was extracted with water and EA and the water was removed using MgSO₄. The crude product was purified by silica gel column chromatography (EA:Hex = 1:4 by volume) to give 1-(6-methoxy-2,4-bis (methoxymethoxy)-3-(3-methylbut-2-en-1-yl)phenyl)ethan-1-one (Compound E) 24.605 g (yield 93.6%).

¹H NMR (400 MHz, chloroform-d) δ6.53 (s, 1H), 5.19 (s, 2H), 5.13 (td, J = 7.7, 6.7, 3.6 Hz, 1H), 4.89 (s, 2H), 3.78 (s, 3H), 3.46 (d, J = 4.5 Hz, 6H), 3.29 (d, J = 6.8 Hz, 2H), 2.48 (s, 3H), 1.73 (s, 3H), 1.64 (s, 3H).

### Synthesis Example 2

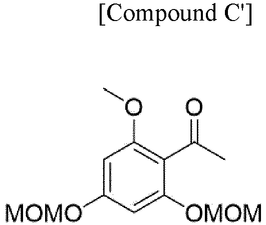

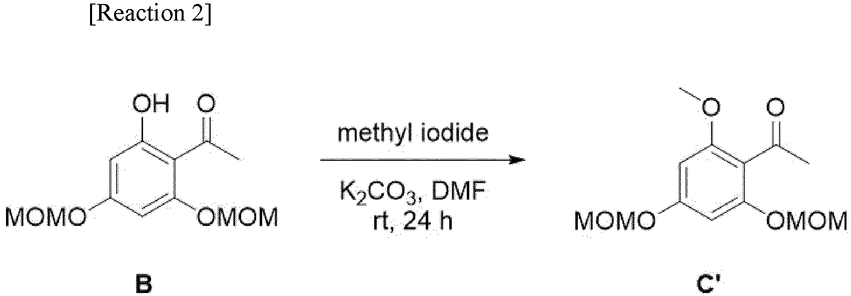

### (i) Synthesis of Compound C'

18.1 g (0.128 mol) of methyl iodide and 17.636 g (0.128 mol) of K₂CO₃ Kwas added to 10.9 g (42.537 mmol) of the obtained compound B in 131 ml of DMF, and the inside of the flask was quenched with Ar. The reaction mixture was then stirred at room temperature for 24 hours. The product is extracted with water and EA and the water is removed with MgSO₄. The product was purified separately by silica gel column chromatography (EA:Hex = 1:4 by volume) to give 8.930 g of 1-(2-methoxy-4,6-bis(methoxymethoxy)phenyl)ethan-1-one (compound C') in 77.7% yield.

¹H NMR (400 MHz, chloroform-d) δ6.44 (d, J = 1.9 Hz, 1H), 6.29 (d, J = 1.8 Hz, 1H), 5.13 (d, J = 9.8 Hz, 4H), 3.77 (s, 3H), 3.45 (dd, J = 8.9, 0.6 Hz, 6H), 2.46 (d, J = 0.6 Hz, 3H).

### Synthesis Example 3

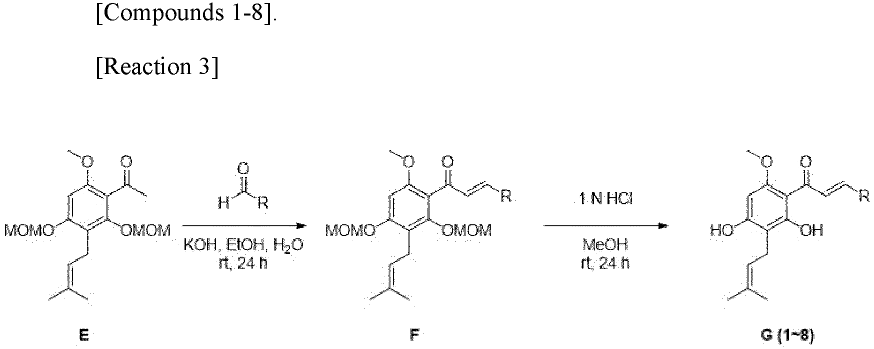

### (i) Synthesis of compound F0

500.0 mg (1.478 mmol) of 1-(6-Methoxy-2,4-bis(methoxymethoxy)-3-(3-methylbut-2-en-1-yl)phenyl)ethan-1-one (Compound E) and 270.1 mg (1.625 mmol) of 4-(methoxymethoxy)benzaldehyde were dissolved in 8.6 ml of ethanol. After the solution was cooled to 0°C, 4.145 g (73.877 mmol) of KOH and 5.3 ml of H₂O were added and the flask was quenched with Ar. The mixture was then stirred at room temperature for 24 hours. The resulting compound was extracted with EA and purified separately by silica gel column chromatography (EA:Hex = 1:3 volume ratio) to give (E)-1-(6-methoxy-2,4-bis(methoxymethoxy)-3-(3-methylbut-2-en-1-yl)phenyl)-3-(4-(methoxymethoxy)phenyl)prop-2-en-1-one (Compound F0) 443.7 mg (yield 61.7%).

### (ii) Synthesis of XN

443.7 mg (0.912 mmol) of the obtained compound F0 was dissolved in 110 ml of methanol and 12.1 ml of an aqueous solution of 6N HCl was added. The flask was quenched with Ar and stirred at room temperature for 24 hours. The obtained product was extracted with EA and then purified by silica gel column chromatography (EA:Hex = 1:2 volume ratio) to give (E)-1-(2,4-dihydroxy-6-methoxy-3-(3-methylbut-2-en-1-yl)phenyl)-3-(4-hydroxyphenyl)prop-2-en-1-one (XN) 321.0 mg (yield 99.3%).

¹H NMR (400 MHz, methanol-d4) δ7.75 (d, J=14.7 Hz, 1H), 7.62 (d, J=14.9 Hz, 1H), 7.46 (d, J=9.6 Hz, 2H), 6.80(d, J=7.5 Hz, 2H), 5.97(s, 1H), 5.17(s, 1H), 3.85(s, 3H), 3.20(d, J=7.2 Hz, 2H), 1.73(s, 3H), 1.62(s, 3H).

### (iii) Synthesis of Compound F1

328.1 mg (1.773 mmol) of 4-Bromobenzaldehyde was synthesized by referring to step (i) of Synthesis Example 3 to give 654.5 mg of (E)-3-(4-bromophenyl)-1-(6-methoxy-2,4-bis(methoxymethoxy)-3-(3-methylbut-2-en-1-yl)phenyl)prop-2-en-1-one (compound F1) (87.6% yield).

### (iv) Synthesis of Compound 1

91.7 mg of ((E)-3-(4-bromophenyl)-1-(2,4-dihydroxy-6-methoxy-3-(3-methylbut-2-en-1-yl)phenyl)prop-2-en-1-one (Compound 1)(yield 17.0%) was synthesized from 654.5 mg (1.295 mmol) of the obtained compound F1 by referring to step (ii) of Synthesis Example 3.

¹H NMR (400 MHz, methanol-d4) δ 7.55 (d, J = 10.5 Hz, 2H), 7.41 (d, J = 8.5 Hz, 2H), 6.11 (s, 1H), 5.38 (dd, J = 12.5, 2.2 Hz, 1H), 5.12 (t, J = 8.3 Hz, 1H), 3.77 (s, 3H), 3.21 (t, J = 6.9 Hz, 2H), 2.92 (dd, J = 16.7, 12.5 Hz, 1H), 2.72 (dd, J = 16.6, 3.2 Hz, 1H), 1.58 (d, J = 16.5 Hz, 6H).

### (v) Synthesis of Compound F2

502.2 mg (81.6% yield) of (E)-3-(furan-2-yl)-1-(6-methoxy-2,4-bis(methoxymethoxy)-3-(3-methylbut-2-en-1-yl)phenyl)prop-2-en-1-one (Compound F2) was synthesized from 328.1 mg (1.773 mmol) of furan-2-carbaldehyde by referring to step (I) of Synthesis Example 3.

### (vi) Synthesis of Compound 2

325.1 mg of (E)-1-(2,4-dihydroxy-6-methoxy-3-(3-methylbut-2-en-1-yl)phenyl)-3-(furan-2-yl)prop-2-en-1-one (Compound 2) (82.1% yield) was synthesized from the obtained compound F2 502.2 mg (1.206 mmol) by referring to step (ii) of Synthetic Example 3.

¹H NMR (400 MHz, chloroform-d) δ14.66 (s, 1H), 7.78 (d, J=14.6 Hz, 1H), 7.57 (d, J=15.0 Hz, 1H), 7.50 (s, 1H), 6.65 (d, J=2.6 Hz, 1H), 6.49 (dd, J=3.3, 1.7 Hz, 1H), 6.18 (s, 1H), 5.92 (s, 1H), 3.89 (s, 3H), 3.39 (d, J=7.2 Hz, 2H), 1.79 (d, J=21.9 Hz, 6H).

### (vii) Synthesis of Compound F3

Using 68.15 mg (0.709 mmol) of furan-3-carbaldehyde, 47.2 mg (19.2% yield) of (E)-3-(furan-3-yl)-1-(6-methoxy-2,4-bis(methoxymethoxy)-3-(3-methylbut-2-en-1-yl) phenyl)prop-2-en-1-one (compound F3) was obtained by referring to step (ii) of Synthetic Example 3.

### (viii) Synthesis of Compound 3

Using the obtained compound F3 47.2 mg (0.113 mmol), (E)-1-(2,4-dihydroxy-6-methoxy-3-(3-methylbut-2-en-1-yl)phenyl)-3-(furan-3-yl)prop-2-en-1-one (Compound 3) 18.2 mg (48.9% yield) was synthesized by referring to step (ii) of Synthetic Example 3.

¹H NMR (400 MHz, methanol-d4) δ 7.77 (s, 1H), 7.52 (s, 1H), 7.22 (d, J = 12.7 Hz, 1H), 6.72 (s, 1H), 6.64 (d, J = 15.8 Hz, 1H), 6.07 (s, 1H), 3.66 (s, 3H), 2.58 (t, J = 6.8 Hz, 2H), 1.73 (t, J = 6.8 Hz, 2H), 1.20 (s, 6H).

### (ix) Synthesis of Compound F4

Using 5-Methylthiophene-2-carbaldehyde 134.23 mg (1.064 mmol), (E)-1-(6-methoxy-2,4-bis(methoxymethoxy)-3-(3-methylbut-2-en-1-yl)phenyl)-3-(5-methylthiophen-2-yl)prop-2-en-1-one (Compound F4) 294.8 mg (74.5% yield) was synthesized by referring to step (i) of Synthesis Example 3.

### (x) Synthesis of Compound 4

Using the obtained compound F4 294.8 mg (0.660 mmol), (E)-1-(2,4-dihydroxy-6-methoxy-3-(3-methylbut-2-en-1-yl)phenyl)-3-(5-methylthiophen-2-yl)prop-2-ene-1-one (compound 4) was obtained in 38.3 mg (yield 16.2%) by referring to step (ii) of Synthetic Example 3 to give.

¹H NMR (400 MHz, chloroform-d) δ 14.68 (s, 1H), 7.84 (d, J = 15.0 Hz, 1H), 7.60 (d, J = 14.8 Hz, 1H), 7.09 (d, J = 3.5 Hz, 1H), 6.72 (d, J = 3.5 Hz, 1H), 6.15 (s, 1H), 5.92 (s, 1H), 3.88 (s, 3H), 3.39 (d, J = 7.2 Hz, 2H), 2.51 (s, 3H), 1.79 (d, J = 21.3 Hz, 6H).

### (xi) Synthesis of Compound F5

Using 110.77 mg (0.709 mmol) of 1-naphthaldehyde, referring to step (i) of Synthesis Example 3, 276.1 mg (98.0% yield) of (E)-1-(6-methoxy-2,4-bis (methoxymethoxy)-3-(3-methylbut-2-en-1-yl)phenyl)-3-(naphthalen-1-yl)prop-2-en-1-one (Compound F5) was obtained.

### (xii) Synthesis of Compound 5

Using the obtained compound F5 276.1 mg (0.579 mmol), (E)-1-(2,4-dihydroxy-6-methoxy-3-(3-methylbut-2-en-1-yl)phenyl)-3-(naphthalen-1-yl)prop-2-en-1-one (compound 5) was obtained 15.7 mg (yield 7.0%), referring to (ii) of synthesis example 3.

¹H NMR (400 MHz, chloroform-d) δ 8.46 (d, J = 15.4 Hz, 1H), 8.24 (s, 1H), 7.88 - 7.78 (m, 3H), 7.74 (d, J = 7.2 Hz, 1H), 7.55 - 7.38 (m, 3H), 5.91 (s, 1H), 5.18 (t, J = 6.9 Hz, 1H), 3.80 (s, 3H), 3.24 (d, J = 6.9 Hz, 2H), 1.71 (s, 3H), 1.61 (s, 3H).

### (xiii) Synthesis of Compound F6

Using 255.4 mg (1.625 mmol) of quinolin-8-carbaldehyde as reference (I) of Synthesis Example 3, (E)-1-(6-methoxy-2,4-bis(methoxymethoxy)-3-(3-methylbut-2-en-1-yl)phenyl)-3-(quinolin-8-yl)prop-2-en-1-one (compound F6) was obtained 590.1 mg (83.6% yield).

### (xiv) Synthesis of Compound 6

Using the obtained compound F6 590.1 mg (1.236 mmol), (E)-1-(2,4-dihydroxy-6-methoxy-3-(3-methylbut-2-en-1-yl)phenyl)-3-(quinolin-8-yl)prop-2-en-1-one (compound 6) 156.8 mg (32.6% yield) was synthesized by referring to step (ii) of Synthetic Example.3

¹H NMR (400 MHz, chloroform-d) δ 14.70 (s, 1H), 9.06 - 8.91 (m, 2H), 8.25 (dd, J = 15.6, 1.7 Hz, 1H), 8.22 - 8.13 (m, 1H), 8.05 (d, J = 7.1 Hz, 1H), 7.85 (d, J = 8.0 Hz, 1H), 7.58 (t, J = 7.7 Hz, 1H), 7.45 (dd, J = 8.7, 5.5 Hz, 1H), 6.35 (s, 1H), 5.30 (t, J = 6.7 Hz, 1H), 3.89 (s, 3H), 3.41 (d, J = 7.0 Hz, 2H), 1.79 (d, J = 21.3 Hz, 6H).

### (xv) Synthesis of Compound F7

Using benzofuran-2-carbaldehyde 111.4 mg (0.762 mmol), (E)-3-(benzofuran-2-yl)-1-(6-methoxy-2,4-bis(methoxymethoxy)-3-(3-methylbut-2-en-1-yl)phenyl)prop-2-en-1-one (compound F7) 256.8 mg (yield 86.6%) was synthesized by referring to step (i) of Synthesis Example 3.

### (xvi) Synthesis of Compound 7

Using the obtained compound F7 256.8 mg (0.550 mmol), (E)-3-(benzofuran-2-yl)-1-(2,4-dihydroxy-6-methoxy-3-(3-methylbut-2-en-1-yl)phenyl)prop-2-en-1-one (compound 7) 74.4 mg (35.7% yield) was synthesized by referring to step (ii) of Synthetic Example 3.

¹H NMR (400 MHz, DMSO-d6) δ14.44(s, 1H), 10.65(s, 1H), 7.91(d, J=15.7 Hz, 1H), 7.74-7.57(m, 3H), 7.44-7.33(m, 2H), 7.26(t, J=7.5 Hz, 1H), 6.08(s, 1H), 3.90(s, 3H), 2.47-2.37(m, 2H), 1.53-1.44(m, 2H), 1.09(s, 6H).

### (xvii) Synthesis of Compound F8

Using terephthalaldehyde 89.18 mg (0.665 mmol), (2E,2'E)-3,3'-(1,4-phenylene)bis(1-(6-methoxy-2,4-bis(methoxymethoxy)-3-(3-methylbut-2-en-1-yl)phenyl)prop-2-en-1-one) (Compound F8) 501.0 mg (43.8% yield) was synthesized by referring to step (i) of Synthetic Example 3.

### (xviii) Synthesis of Compound 8

Using the obtained compound F8 101.8 mg (0.131 mmol), 45.1 mg (60.4% yield) of (2E,2'E)-3,3'-(1,4-phenylene)bis(1-(2,4-dihydroxy-6-methoxy-3-(3-methylbut-2-en-1-yl)phenyl)prop-2-en-1-one) (compound 8) was obtained, referring to (ii) of Synthetic Example 3.

¹H NMR (400 MHz, DMSO-d6) δ9.76(s, 2H), 7.62(s, 4H), 7.14(d, J=15.2 Hz, 2H), 6.93(d, J=15.9 Hz,2H), 6.08(s,2H), 3.57(s,6H), 1.66(t, J=6.7 Hz,4H), 1.12(s, 12H).

### Synthesis Example 4

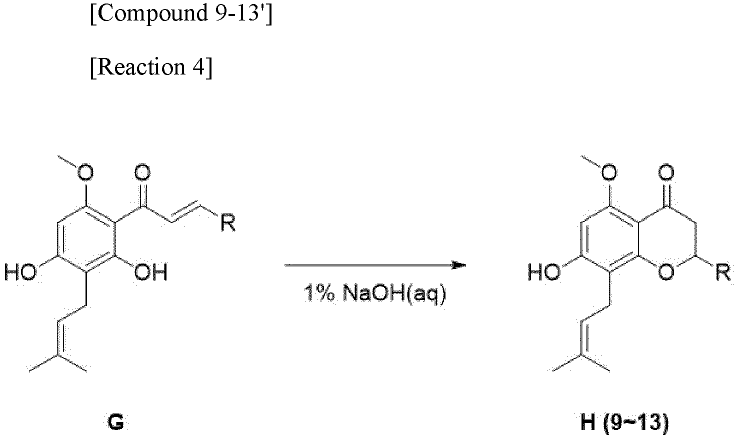

### (i) Synthesis of IX

112 ml of 1% aqueous NaOH solution was added to (E)-1-(2,4-dihydroxy-6-methoxy-3-(3-methylbut-2-en-1-yl)phenyl)-3-(4-hydroxyphenyl)prop-2-en-1-one(XN) 200.0 mg (0.135 mmol), followed by stirring at room temperature for 2 hr. The obtained product was extracted with EA under acidic conditions using 1 N HCl aqueous solution, followed by purification by silica gel column chromatography (EA:Hex=1:1 volume ratio) to give 7-hydroxy-2-(4-hydroxyphenyl)-5-methoxy-8-(3-methylbut-2-en-1-yl)chroman-4-one (IX) 183.1 mg (91.6% yield).

¹H NMR (400 MHz, methanol-d4) δ7.29 (d, J=12.3 Hz, 2H), 6.79 (d, J=11.8 Hz, 2H), 6.09 (s, 1H), 5.26 (d, J=15.5 Hz, 1H), 5.11(t, J=7.2Hz, 1H), 3.78(s, 3H), 3.18(d, J=7.2Hz, 1H), 2.96(dd, J=16.7, 12.7Hz, 1H), 1.59(s, 3H), 1.54(s, 3H).

### (ii) Synthesis of Compound 9

Using 64.7 mg (0.155 mmol) of compound 1, 2-(4-bromophenyl)-7-hydroxy-5-methoxy-8-(3-methylbut-2-en-1-yl)chroman-4-one (Compound 9) 55.4 mg (85.6% yield) was synthesized by referring to step (i) of Synthetic Example 4.

¹H NMR (400 MHz, methanol-d4) δ 7.55 (d, J = 10.5 Hz, 2H), 7.41 (d, J = 8.5 Hz, 2H), 6.11 (s, 1H), 5.38 (d, J = 12.5 Hz, 1H), 5.12 (t, J = 8.3 Hz, 1H), 3.77 (s, 3H), 3.21 (t, J = 7.5 Hz, 2H), 2.92 (dd, J = 16.7, 12.5 Hz, 1H), 2.72 (d, J = 16.6 Hz, 1H), 1.58 (d, J = 16.5 Hz, 6H).

### (iii) Synthesis of Compound 10

Using 108.1 mg (0.329 mmol) of Compound 2, 2-(furan-2-yl)-7-hydroxy-5-methoxy-8-(3-methylbut-2-en-1-yl)chroman-4-one (Compound 10) 100.3 mg (92.8% yield) was synthesized by referring to step (i) of Synthetic Example 4.

¹H NMR (400 MHz, methanol-d4) δ7.52 (s, 1H), 6.45 (d, J=3.8 Hz, 1H), 6.44-6.36 (m, 1H), 6.09 (s, 1H), 5.44 (dd, J=14.5, 3.5Hz, 1H), 5.09(t, J=7.3 Hz, 1H), 3.77(s, 3H), 3.16(d, J=7.3 Hz, 2H), 3.13-3.04(m, 1H), 2.00(d, J=9.4 Hz, 2H), 1.59(s, 6H).

### (iv) Synthesis of Compound 11

Using 36.0 mg (0.100 mmol) of Compound 4, 7-hydroxy-5-methoxy-8-(3-methylbut-2-en-1-yl)-2-(5-methylthiophen-2-yl)chroman-4-one (Compound 11) 25.1 mg (69.7% yield) was synthesized by referring to step (i) of Synthetic Example 4.

¹H NMR (400 MHz, methanol-d4) δ 6.87 (d, J = 3.4 Hz, 1H), 6.63 (d, J = 2.4 Hz, 1H), 6.08 (s, 1H), 5.54 (dd, J = 10.1, 3.2 Hz, 1H), 5.13 (t, J = 7.8 Hz, 1H), 3.76 (s, 3H), 3.19 (d, J = 7.2 Hz, 2H), 3.03 - 2.89 (m, 1H), 2.84 (dd, J = 16.6, 3.9 Hz, 1H), 2.44 (s, 3H), 1.60 (d, J = 4.6 Hz, 6H).

### (v) Synthesis of Compound 12

Using 110.4 mg (0.283 mmol) of Compound 6, 7-hydroxy-5-methoxy-8-(3-methylbut-2-en-1-yl)-2-(quinolin-8-yl)chroman-4-one (compound 12) 21.5 mg (19.5% yield) was synthesized by referring to step (i) of Synthetic Example 4.

¹H NMR (400 MHz, methanol-d4) δ 8.87 (dd, J=4.2, 1.7 Hz, 1H), 8.33 (dd, J= 8.3, 1.7 Hz, 1H), 8.06 (d, J=7.2 Hz, 1H), 7.92 (d, J=8.1 Hz, 1H), 7.65 (t, J= 7.3 Hz, 1H), 7.52 (dd, J=8.3, 4.2 Hz, 1H), 6.51 (dd, J = 12.7, 2.9 Hz, 1H), 6.15 (s, 1H), 5.17 (t, J=7.2 Hz, 1H), 3.81 (s, 3H), 3.07 (dd, J=16.7, 3.0 Hz, 1H), 2.88 (dd, J=16.7, 13.1 Hz, 1H), 1.55 (d, J=14.9 Hz, 6H).

### (vi) Synthesis of Compound 13

Using 110.6 mg (0.291 mmol) of (E)-1-(2,4-Dihydroxy-6-methoxy-3-(3-methylbut-2-en-1-yl)phenyl)-3-(4-isopropylphenyl)prop-2-en-1-one, 7-hydroxy-2-(4-isopropylphenyl)-5-methoxy-8-(3-methylbut-2-en-1-yl)chroman-4-one (compound 13) 65.3 mg (yield 59.0%) was synthesized by referring to step (i) of Synthetic Example 4.

¹H NMR (400 MHz, methanol-d4) δ 7.41 (d, J = 8.1 Hz, 2H), 7.26 (d, J = 8.1 Hz, 2H), 6.11 (s, 1H), 5.36 (dd, J = 12.8, 2.6 Hz, 1H), 3.78 (s, 3H), 3.20 (s, 1H), 2.69 (dd, J = 16.8, 2.8 Hz, 1H), 2.62-2.48 (m, 2H), 1.60 (td, J = 18.6, 17.1, 6.6 Hz, 2H), 1.23 (d, J = 6.9 Hz, 6H), 1.11 (s, 6H).

### Synthesis Example 5

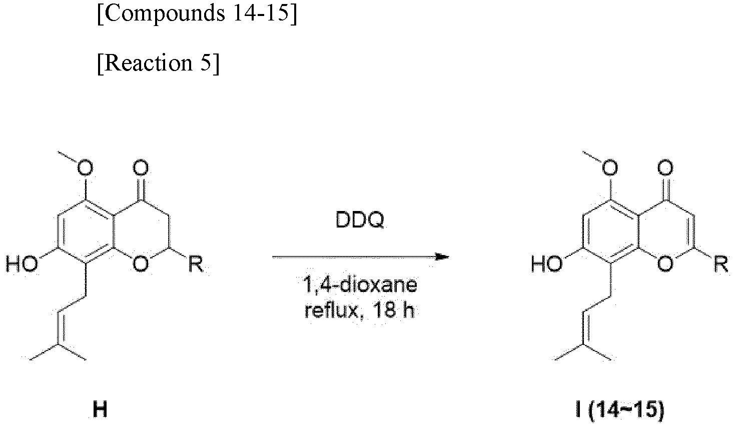

### (i) Synthesis of Compound 14

50.0 mg (0.141 mmol) of 7-hydroxy-2-(4-hydroxyphenyl)-5-methoxy-8-(3-methylbut-2-en-1-yl)chroman-4-one (IX) and 96.08 mg (0.423 mmol) of 2,3-Dichloro-5,6-dicyano-p-benzoquinone (DDQ) was dissolved in 1.69 ml of 1,4-dioxane. The solution was stirred at reflux for 18 hours. The obtained product was purified by silica gel column chromatography (MC:MeOH = 20:1 volume ratio) to give 10.1 mg (20.3% yield) of 7-hydroxy-2-(4-hydroxyphenyl)-5-methoxy-8-(3-methylbut-2-en-1-yl)-4H-chromen-4-one (compound 14).

¹H NMR (400 MHz, methanol-d4) δ 7.81 (d, J = 15.1 Hz, 2H), 6.92 (d, J = 9.1 Hz, 2H), 6.54 (s, 1H), 6.43 (s, 1H), 5.75 (d, J = 10.0 Hz, 1H), 3.87 (s, 3H), 2.00 (d, J = 9.7 Hz, 1H), 1.47 (s, 6H).

### (ii) Synthesis of Compound 15

Using 120.2 mg (0.336 mmol) of Compound 10, 2-(furan-2-yl)-7-hydroxy-5-methoxy-8-(3-methylbut-2-en-1-yl)-4H-chromen-4-one (Compound 15) 17.2 mg (yield 14.4%) was synthesized by referring to step (i) of Synthetic Example 5.

¹H NMR (400 MHz, methanol-d4) δ 7.79 (d, J = 8.7 Hz, 2H), 6.88 (dd, J = 19.4, 9.4 Hz, 3H), 6.53 (s, 1H), 6.41 (s, 1H), 5.79 - 5.69 (m, 1H), 3.86 (s, 3H), 2.00 (q, J = 12.2, 10.5 Hz, 1H), 1.47 (s, 6H).

### Synthesis Example 6

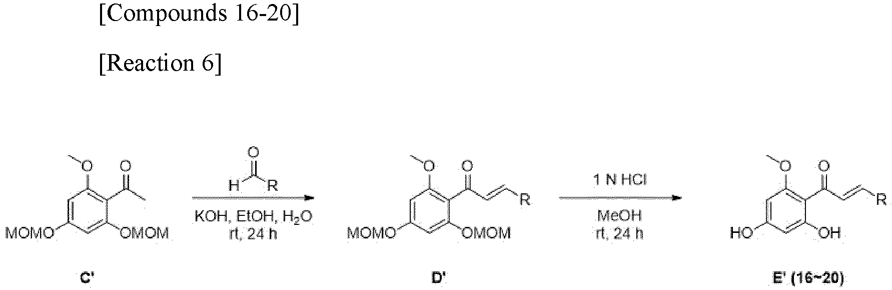

### (i) Synthesis of compound D'0

1-(2-Methoxy-4,6-bis(methoxymethoxy)phenyl)ethan-1-one (compound C') was synthesized using 500 mg (1.850 mmol) of 1-(2-methoxy-4,6-bis (methoxymethoxy)phenyl)ethan-1-one (compound C') by referring to step (i) of Synthesis Example 3 to give (E)-3-(4-hydroxyphenyl)-1-(2-methoxy-4,6-bis(methoxymethoxy)phenyl)prop-2-ene-1-one (compound D') 335.9 mg (43.4% yield).

### (ii) Synthesis of Helichrysanthemum

Using 235.9 mg (0.564 mmol) of (E)-1-(2-methoxy-4,6-bis(methoxymethoxy) phenyl)-3-(4-(methoxymethoxy)phenyl)prop-2-en-1-one (Compound D'), (E)-1-(2,4-dihydroxy-6-methoxyphenyl)-3-(4-hydroxyphenyl)prop-2-en-1-one (helicrycetin) 110.1 mg (yield 68.2%) was synthesized by referring to step (ii) in Example 3.

¹H NMR (400 MHz, DMSO-d6) δ 13.93 (s, 1H), 7.61 (d, J = 8.4 Hz, 2H), 7.53 (d, J = 8.7 Hz, 2H), 6.79 (d, J = 8.6 Hz, 2H), 5.96 (d, J = 2.1 Hz, 1H), 5.86 (d, J = 2.1 Hz, 1H), 3.83 (s, 3H).

### (iii) Synthesis of Compound D'1

Using 410.7mg (2.220mmol) of 4-Bromobenzaldehyde, (E)-3-(4-bromophenyl) -1-(2-methoxy-4,6-bis(methoxymethoxy)phenyl)prop-2-en-1-one(compound D'1) 718.3 mg (88.8% yield) was synthesized by referring to step (i) of Synthesis Example 6.

### (iv) Synthesis of Compound 16

Using the obtained compound D'1 718.3 mg (1.643 mmol), 458.5 mg (yield 79.9%) of ((E)-3-(4-bromophenyl)-1-(2,4-dihydroxy-6-methoxy-3-(3-methylbut-2-en-1-yl)phenyl)prop-2-en-1-one (compound 16) was synthesized by referring to step (ii) of Synthesis Example 6

¹H NMR (400 MHz, chloroform-d) δ 14.05 (s, 1H), 7.84 (d, J=15.4 Hz, 1H), 7.68 (d, J=15.6 Hz, 1H), 7.52 (s, 2H), 7.45 (d, J=8.5 Hz, 2H), 6.02 (d, J=2.3 Hz, 1H), 5.94 (d, J=2.3 Hz, 1H), 5.30 (s, 1H), 3.92 (s, 3H).

### (v) Synthesis of Compound D'2

Using 303.4 mg (2.405 mmol) of 5-Methylthiophene-2-carbaldehyde, (E)-1-(2-methoxy-4,6-bis(methoxymethoxy)phenyl)-3-(5-methylthiophen-2-yl)prop-2-en-1-one (compound D'2) 265.2 mg (37.9% yield) was synthesized by referring to step (i) of Synthesis Example 6

### (vi) Synthesis of Compound 17

Using 265.2 mg (0.701 mmol) of the obtained compound D'2, (E)-1-(2,4-dihydroxy-6-methoxyphenyl)-3-(5-methylthiophen-2-yl)prop-2-en-1-one (compound 17) 198.2 mg (97.4% yield) was synthesized by referring to step (ii) of Synthesis Example 6.

¹H NMR (400 MHz, methanol-d4) δ 7.74 (d, J = 15.0 Hz, 1H), 7.56 (d, J = 15.2 Hz, 1H), 7.11 (d, J = 3.6 Hz, 1H), 6.74 (d, J = 3.5 Hz, 1H), 5.95 (d, J = 2.1 Hz, 1H), 5.89 (d, J = 2.1 Hz, 1H), 3.87 (s, 3H), 2.47 (s, 3H).

### (vii) Synthesis of Compound D'3

Using 346.7 mg (2.220 mmol) of 1-Naphthaldehyde, (E)-1-(2-methoxy-4,6-bis(methoxymethoxy)phenyl)-3-(naphthalen-2-yl)prop-2-en-1-one(compound D'3) 693.4 mg (91.8% yield) was synthesized by referring to step (i) of Synthesis Example 6.

### (viii) Synthesis of Compound 18

Using 693.4 mg (1.698 mmol) of the obtained compound D'3, (E)-1-(2,4-dihydroxy-6-methoxyphenyl)-3-(naphthalen-2-yl)prop-2-en-1-one (compound 18) 523.4 mg (96.2% yield) was synthesized by referring to step (ii) of Synthesis Example 6.

¹H NMR (400 MHz, chloroform-d) δ 14.17 (s, 1H), 7.97 (t, J = 6.1 Hz, 3H), 7.92-7.79 (m, 3H), 7.75 (dd, J = 8.7, 1.5 Hz, 1H), 7.55 - 7.46 (m, 2H), 6.03 (d, J = 2.4 Hz, 1H), 5.96 (d, J = 1.9 Hz, 1H), 3.96 (s, 3H).

### (ix) Synthesis of Compound D'4

Using 324.43 mg (2.220 mmol) of Benzofuran-2-carbaldehyde, (E)-3-(benzofuran-2-yl)-1-(2-methoxy-4,6-bis(methoxymethoxy)phenyl)prop-2-en-1-one (compound D'4) 536.8 mg (72.8% yield) was synthesized by referring to step (i) of Synthesis Example 6.

### (x) Synthesis of Compound 19

Using 536.8 mg (1.347 mmol) of the obtained compound D'4, (E)-3-(benzofuran-2-yl)-1-(2,4-dihydroxy-6-methoxyphenyl)prop-2-en-1-one (compound 19) 410.9 mg (98.3% yield) was synthesized by referring to step (ii) of Synthesis Example 6.

¹H NMR (400 MHz, chloroform-d) δ 14.13 (s, 1H), 7.98 (d, J = 15.4 Hz, 1H), 7.66 (d, J = 15.3 Hz, 1H), 7.59 (d, J = 7.8 Hz, 1H), 7.50 (d, J = 8.3 Hz, 1H), 7.35 (d, J = 8.8 Hz, 1H), 7.22 (s, 1H), 6.99 (s, 1H), 5.99 (dd, J = 25.6, 2.3 Hz, 2H), 3.96 (s, 3H).

### (xi) Synthesis of Compound D'5

Using 329.0 mg (2.220 mmol) of 4-isopropylbenzaldehyde, (E)-3-(4-isopropylphenyl)-1-(2-methoxy-4,6-bis(methoxymethoxy)phenyl)prop-2-en-1-one (compound D'5) 709.0 mg (yield 95.7%) was synthesized by referring to step (i) of Synthesis Example 6.

### (xii) Synthesis of Compound 20

Using 276.1 mg (0.579 mmol) of the obtained compound D'5, (E)-1-(2,4-dihydroxy-6-methoxyphenyl)-3-(4-isopropylphenyl)prop-2-en-1-one (compound 20) 265.1 mg (54.8% yield) was synthesized by referring to step (ii) of Synthesis Example 6.

¹H NMR (400 MHz, chloroform-d) δ 14.20 (s, 1H), 7.81 (q, J=15.5 Hz, 2H), 7.53 (d, J=8.1 Hz, 2H), 7.27 (s, 3H), 5.98(dd, J=29.5, 2.3 Hz, 2H), 5.33(s, 1H), 3.92(s, 3H), 2.93(hept, J=6.7 Hz, 1H), 1.27(d, J=7.2 Hz, 6H).

### Synthesis Example 7

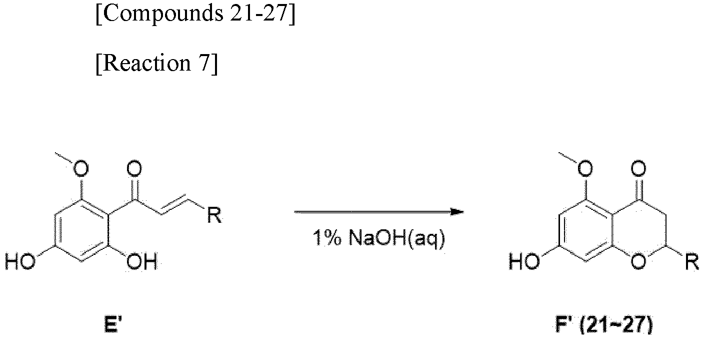

### (i) Synthesis of Compound 21

Using 357.5 mg (1.024 mmol) of the obtained compound 16, 2-(4-bromophenyl)-7-hydroxy-5-methoxychroman-4-one (compound 21) 246.2 mg (68.9% yield) was synthesized by referring to step (i) of Synthesis Example 4.

¹H NMR (400 MHz, methanol-d4) δ 7.54 (d, J = 8.5 Hz, 2H), 7.40 (d, J = 7.9 Hz, 2H), 6.05 (dd, J = 23.5, 1.7 Hz, 2H), 5.40 (dd, J = 12.5, 2.9 Hz, 1H), 3.80 (s, 3H), 3.01 - 2.86 (m, 1H), 2.70 (dd, J = 16.7, 3.1 Hz, 1H).

### (ii) Synthesis of Compound 22

(E) Using (E)-1-(2,4-dihydroxy-6-methoxyphenyl)-3-(furan-2-yl)prop-2-en-1-one 276.4 mg (1.062 mmol), 2-(furan-2-yl)-7-hydroxy-5-methoxychroman-4-one (compound 22) was obtained 59.1 mg (21.4% yield), referring to step (ii) of Synthesis Example 4.

¹H NMR (400 MHz, methanol-d4) δ 7.50 (s, 1H), 6.45 (d, J = 3.3 Hz, 1H), 6.43 - 6.35 (m, 1H), 6.05 (d, J = 1.6 Hz, 1H), 5.94 (d, J = 1.5 Hz, 1H), 5.44 (dd, J = 11.7, 3.2 Hz, 2H), 3.78 (s, 3H), 3.09 (dd, J = 16.7, 11.3 Hz, 1H), 2.76 (dd, J = 16.8, 3.7 Hz, 1H).

### (iii) Synthesis of Compound 23

Using 153.6 mg (0.529 mmol) of the obtained Compound 17, 7-hydroxy-5-methoxy-2-(5-methylthiophen-2-yl)chroman-4-one (Compound 23) 44.8 mg (29.2% yield) was synthesized by referring to step (i) of Synthesis Example 4.

¹H NMR (400 MHz, methanol-d4) δ 6.87 (d, J=3.5 Hz, 1H), 6.63 (d, J=2.5 Hz, 1H), 6.05 (d, J=1.9 Hz, 1H), 5.96 (d, J=2.0Hz, 1H), 5.55 (dd, J=11.5, 3.2Hz, 1H), 3.78 (s, 3H), 3.04-2.91 (m, 1H), 2.82 (dd, J=16.7, 3.5Hz, 1H), 2.43 (s, 3H).

### (iv) Synthesis of Compound 24

Using 438.4 mg (1.369 mmol) of the obtained Compound 18, 7-hydroxy-5-methoxy-2-(naphthalen-2-yl)chroman-4-one (Compound 24) 210.6 mg (48.0% yield). was synthesized by referring to step (i) of Synthesis Example 4 was synthesized by referring to step (i) of Synthesis Example 4.

¹H NMR (400 MHz, DMSO-d6) δ 7.92 (dd, J=23.7, 12.1 Hz, 4H), 7.61 (d, J=11.5 Hz, 1H), 7.54-7.45 (m, 2H), 6.06 (d, J=16.6 Hz, 2H), 5.61 (d, J=12.9 Hz, 1H), 3.72 (s, 3H), 3.06 (dd, J=15.7, 13.1 Hz, 1H), 2.69 (d, J=16.5 Hz, 1H).

### (v) Synthesis of Compound 25

Using 325.9 mg (1.050 mmol) of the obtained compound 19, 2-(benzofuran-2-yl)-7-hydroxy-5-methoxychroman-4-one (compound 25) 121.4 mg (37.3% yield) was synthesized by referring to step (i) of Synthesis Example 4.

¹H NMR (400 MHz, DMSO-d6) δ 7.51 (d, J=7.2 Hz, 1H), 7.45 (d, J=7.9 Hz, 1H), 7.17 (dt, J=14.5, 7.2 Hz, 2H), 6.64(s, 1H), 6.06-5.95(m, 2H), 4.98(dd, J=8.4, 3.7Hz, 1H), 3.75(s, 3H), 3.31(d, J=3.7Hz, 1H), 3.17-3.03(m, 1H).

### (vi) Synthesis of Compound 26

Using (E)-1-(2,4-dihydroxy-6-methoxyphenyl)-3-(4-nitrophenyl)prop-2-en-1-one 45.5 mg (0.144 mmol), 7-hydroxy-5-methoxy-2-(4-nitrophenyl)chroman-4-one (compound 26) was obtained 16.3 mg (32.2% yield), referring to step (i) of Synthesis Example 4.

¹H NMR (400 MHz, DMSO-d6) δ 11.36 (s, 1H), 8.26 (d, J = 8.9 Hz, 2H), 8.10 (d, J = 8.9 Hz, 2H), 6.76 (s, 1H), 6.36 (d, J = 1.4 Hz, 1H), 6.18 (d, J = 1.5 Hz, 1H), 3.83 (s, 3H), 1.22 - 1.05 (m, 2H).

### (vii) Synthesis of Compound 27

Using 194.6 mg (0.623 mmol) of the obtained compound 20, 7-hydroxy-2-(4-isopropylphenyl)-5-methoxychroman-4-one (compound 27) 185.6 mg (yield 95.4%) was synthesized by referring to step (i) of Synthesis Example 4.

¹H NMR (400 MHz, methanol-d4) δ 7.37 (d, J=7.9 Hz, 2H), 7.25 (d, J=7.8 Hz, 2H), 6.04 (dd, J=23.9, 2.1 Hz, 2H), 5.36 (dd, J=12.5, 2.8 Hz, 1H), 3.80 (s, 3H), 3.04-2.83 (m, 2H), 2.67 (dd, J=16.4, 2.8 Hz, 1H), 1.23 (d, J=6.9 Hz, 6H).

### Experimental Example

### Methods

Human multiple myeloma cell lines U266 (ATCC CCL-155), IM-9 (ATCC CCL-159), RPMI 8226 (ATCC TBI-196), and ARH-77 (ATCC CRL-1621) were purchased from the American Type Culture Collection (ATCC, Rockville, MD, USA). Cells were used in RPMI1640 culture medium (Gibco Laboratories) supplemented with 10% heat-inactivated fetal bovine serum (Gibco Laboratories), 1% penicillin/ streptomycin (Thermo Fisher Scientific). Cells were cultured in a thermostat incubator at 37°C in 5% CO₂, and passaged every 3-4 days.

### Animals

A total of 12 male NSG (NOD scid gamma) mice were used. The animals were housed in a semi-specific pathogen free (Semi-SPF) animal house at the Chonnam National University Center for Biomedical Science Convergence at a temperature of 20-24°C, humidity of 40-60%, and a 12-hour light/dark cycle, with 5 animals per cage. Sterile water and feed were provided ad libitum, and all experimental procedures were in accordance with the Animal welfare act and approved by the Institutional Animal Care and Use Committee of Chonnam National University (Approval No.: CNU IACUC-H-2020-36).

### Cell viability analysis

KBB-NX series synthetic derivatives were prepared as a stock of 100 mM in Dimethyl Sulfoxide (DMSO), stored at -20°C and diluted in media. The multiple myeloma cell lines U266, IM-9, RPMI 8226 and ARH-77 were treated with 25 µM, 50 µM and 100 µM equal concentrations of KBB-NX series compounds, suspended in 100 µL of culture medium, dispensed into 96-well plates (5x10⁵ cells/well) and incubated for 24 hours. Afterward, 10 µL of EZ-CyTox was mixed into the culture medium using the Cell Viability Assay Kit (EZ-CyTox, Daeil Lab Service CO., Seoul, Korea) and reacted in the dark at 5% CO₂, 37°C for 1 hour.

EZ-CYTOX was measured using a VERSA Max Micro plate reader (Molecular Devices, Sunnyvale, CA, USA) at an absorbance of 450 nm by reacting with dehydrogenase in the cells to produce an orange-colored, water-soluble formazan. The formula for calculating the percentage of viable cells from cells treated with the test compounds to untreated cells was as follows: *Cell viability (%) = [OD (KBB-NX treated cells)-OD (Blank)] / [OD (cells)- OD (Blank)] x 100

### Cell death assays

Multiple myeloma cells U266, IM-9, RPMI8226, ARH-77 (3 x 10⁶ cells/mL) treated with KBB-NX for 48 hours were suspended in 1 x PBS (phosphate-buffered saline, pH 7.4), followed by several washes with 1 x PBS (phosphate-buffered saline, pH 7. 4) and staining with 5 µL of Annexin V-FITC and 5 µL of propidium iodine (PI) (Sigma-Aldrich, St. Louis, MO) in 500 µL of 1 x PBS (1x10⁶ cells/mL) with suspended cells for 30 min in the dark at room temperature. The sections were then subjected to flow cytometry with a FACSCalibur (Becton Dickinson, Franklin Lakes, NJ, USA) by adding 500 µL of 1X binding buffer and analyzed using the CellQuest software (Becton Dickinson) program.

### Cell cycle analysis

Multiple myeloma cells IM-9 (3 x 10⁶ cells/mL) treated with KBB-NX-16 for 48 hours were collected, washed with 1 x PBS, and fixed with 70% ethanol for 24 hours. Next, 500 µL of 1 x PBS (1 x 10⁶ cells/mL) with cells suspended in 1 x PBS after several washes with 1 x PBS was treated with 0.1% RNase A, 25 µg/mL PI and reacted for 30 minutes in the dark at room temperature. Cell cycles were then analyzed using FACS Calibur and Cell Quest software.

### Measure intracellular free radicals

Multiple myeloma cells IM-9 (3 x 10⁶ cells/mL) treated with KBB-NX-20 for 48 hours were collected and suspended in DCFH-DA (20 µM) (Sigma-Aldrich) solution. The reaction was incubated at 37°C for 30 minutes and analyzed by flow cytometry.

### Western blot analysis

For protein extraction from multiple myeloma cells (1 x 10⁷ cells/ mL) treated with 50 µM of KBB-NX-16 and KBB-NX-20, 50 µL of RIPA solution (50 mM Tris-HCl, pH7.4, 150 mM NaCl, 1% Triton X-100, 0.1% SDS, 1 mM EDTA, 1 mM Na3VO4, 1 mM NaF, protease inhibitor cocktail) was added and reacted at 4°C for 30 min. The protein in the supernatant was then collected by centrifugation at 15,000g for 20 min at 4°C, and the protein concentration was quantified using the Bradford Protein assay kit (BioRad, Hercules, CA). After quantification, 25 µg protein was transferred to a PVDF membrane for 2 h at 300 mA after running 10-12% SDS-PAGE. The protein-transferred membrane was blocked with TBS-T containing 5% non-fat dry milk and reacted with primary antibodies, anti-PHB2 and anti-β-actin antibodies (Cell signaling technology, Danvers, MA) for 18-24 hours at 4°C. After the reaction, the membrane was washed three times at 10 min intervals with TBS-T and then reacted with secondary antibodies for 1 hr, followed by three washes at 10 min intervals with TBS-T again. After washing, the membranes were sensitized by reacting with ECL solution (Amersham, USA) for 1 min, and the presence and amount of protein expression was quantified by comparing the size of the bands.

### Apoptosis-related proteins analysis

Expression of proteins associated with apoptosis was determined using the human apoptosis array kit (R&D systems, MN, USA). Nitrocellulose membranes probed with 35 apoptosis-related antibodies were blocked at room temperature for 1 hour, and then 200 µg of extracted proteins were reacted at 4°C for 12 hours. After the reaction, the detection antibody cocktail was treated for 1 hour, followed by a 30-minute reaction with streptavidin-HRP, and visualized with Chemi Reagent.

### Establishing a Multiple Myeloma Model

### Inject RPMI8226-GFP-FLUC cells into a mouse tail vein

Twelve 6-11 week old NSG mice were injected with 1 x 10⁷ RPMI8226-GFP-FLUC cell line (GenTargetInc., CA, USA) suspended in 100 µL of 1XPBS into the tail vein.

### Monitoring for multiple myeloma

Mice were injected with RPMI8226-GFP-FLUC cells, and 8 days later, cancer formation was determined using an *in vivo* imaging system (IVIS) (NightOWL II LB983 NC100, Berthold, Germany).

### Administration of KBB-NX compounds in multiple myeloma mice

To investigate the effect of KBB-NX-2 in a mouse model of multiple myeloma, the RPMI8226- GFP-FLUC cell line was injected and 2 weeks later, 5 mg/kg of KBB-NX-2 was administered intravenously into the tail vein 2 days per week for 3 cycles. The control group received the same amount of PBS, and the positive control group received the same amount of lenalidomide (Sigma-Aldrich).

### Assessment for initial liver, kidney, and cardiac toxicity

As an initial toxicity evaluation of KBB-NX series synthetic compounds, KBB-NX-16 and KBB-NX-20 were injected intravenously into the femoral vein of rats at a dose of 5mg/kg and blood was collected 3 hours later to test for markers of liver toxicity (marker ALT), nephrotoxicity (BUN, creatinine) and cardiotoxicity (CK-MB, myoglobin).

### Experimental results

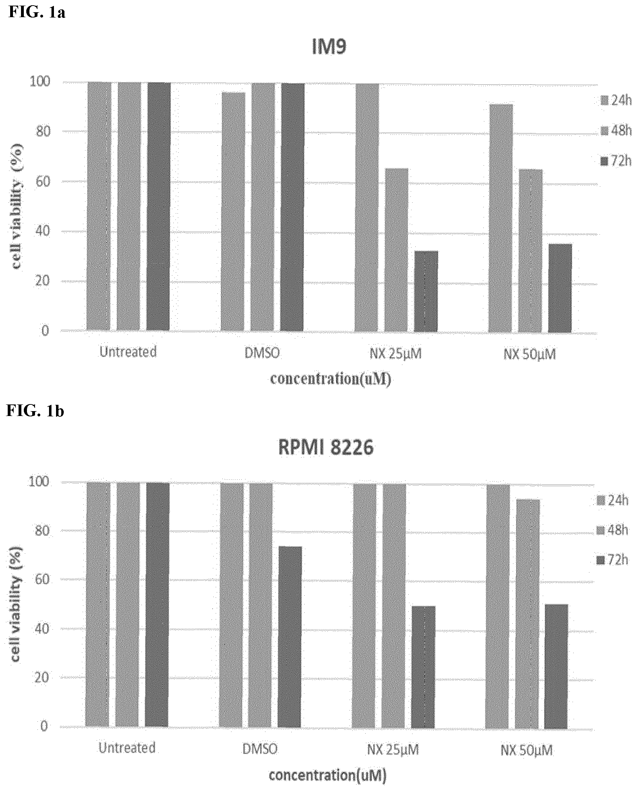

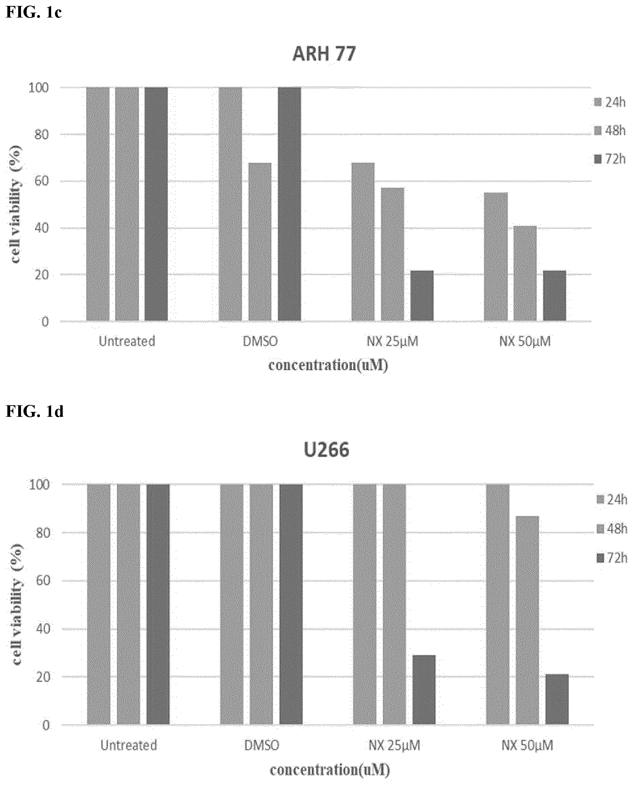

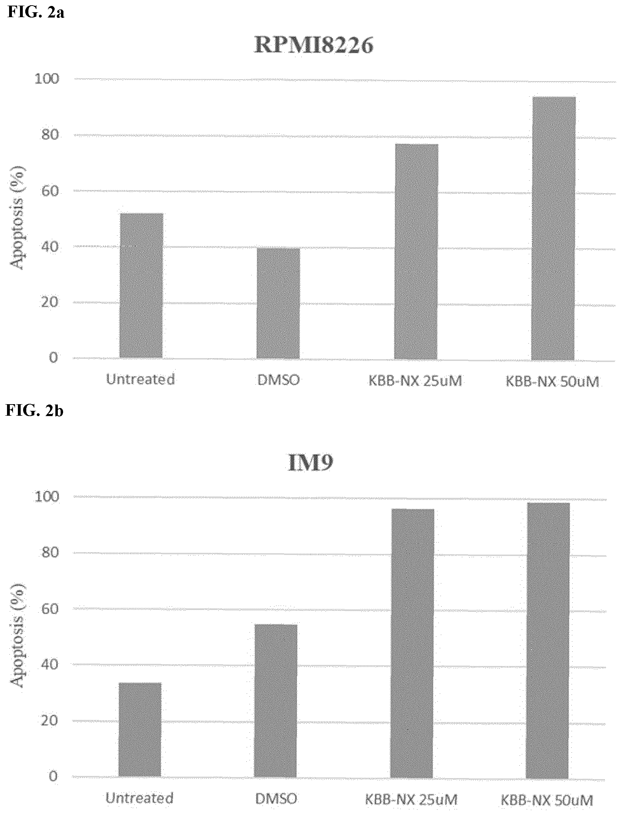

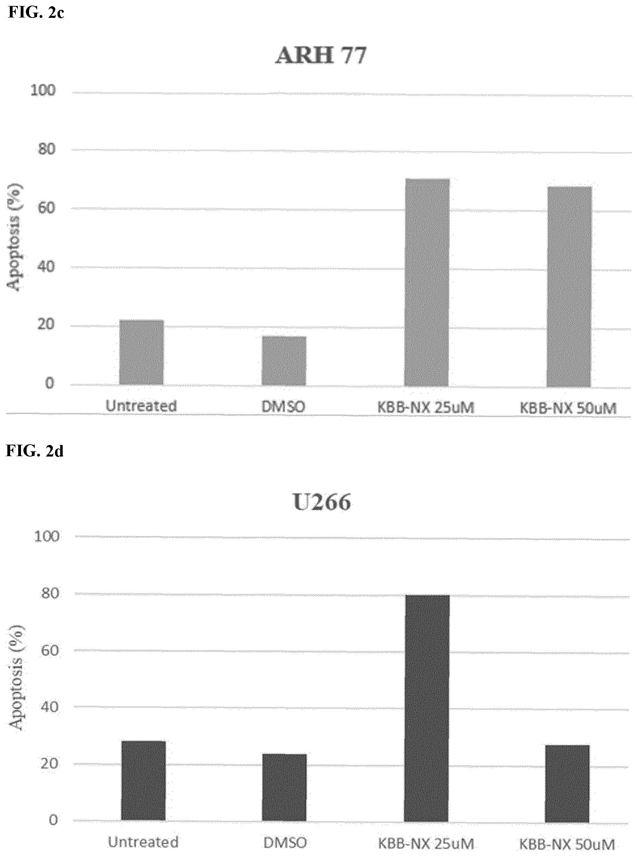

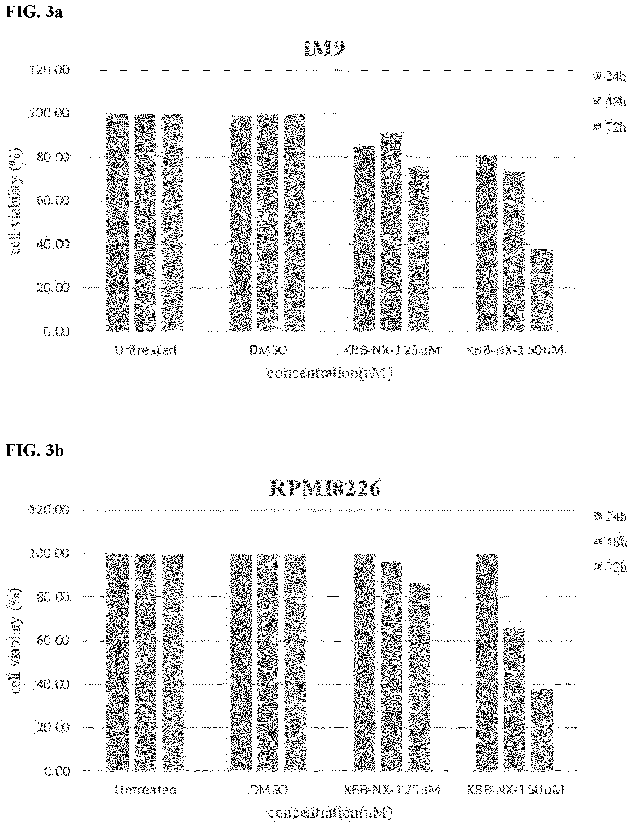

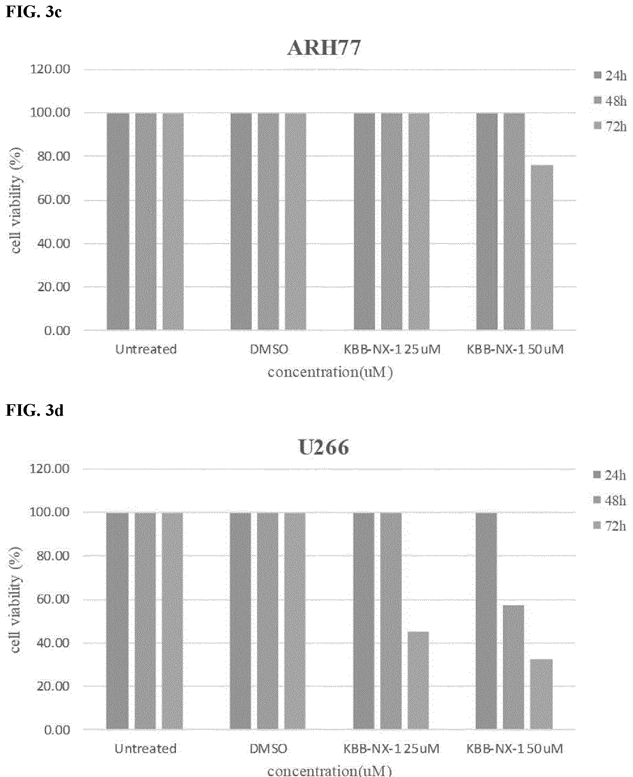

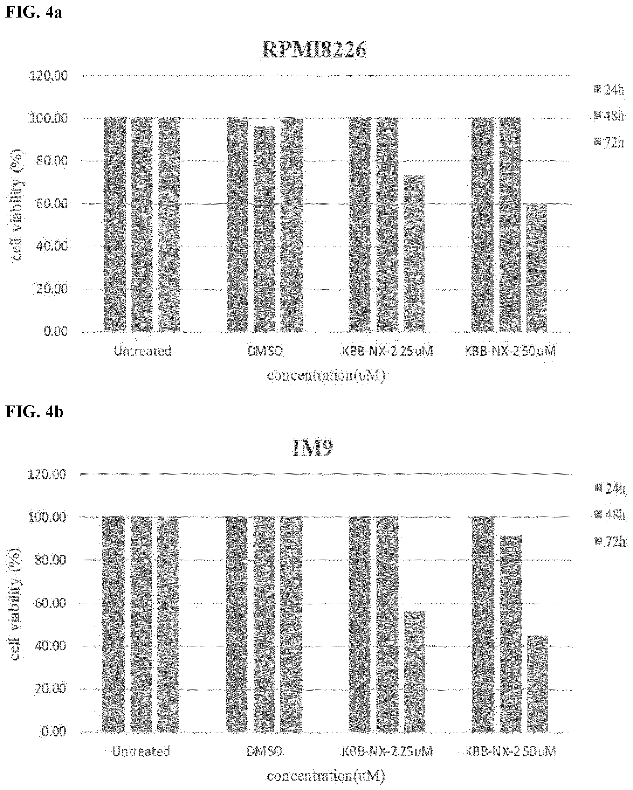

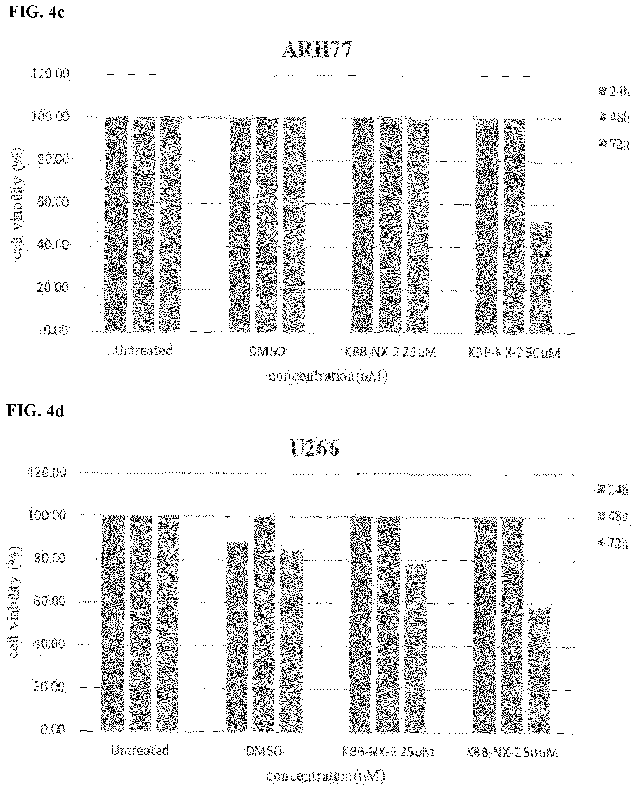

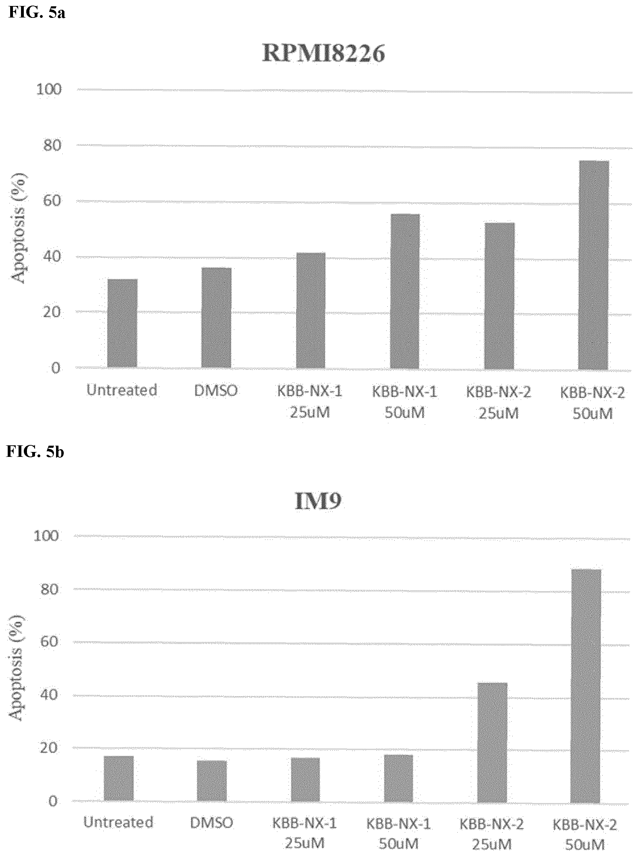

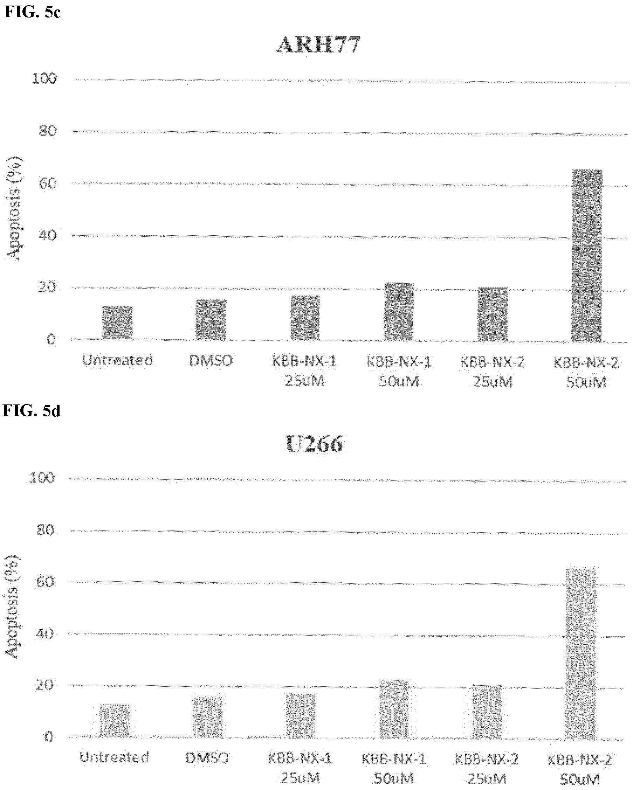

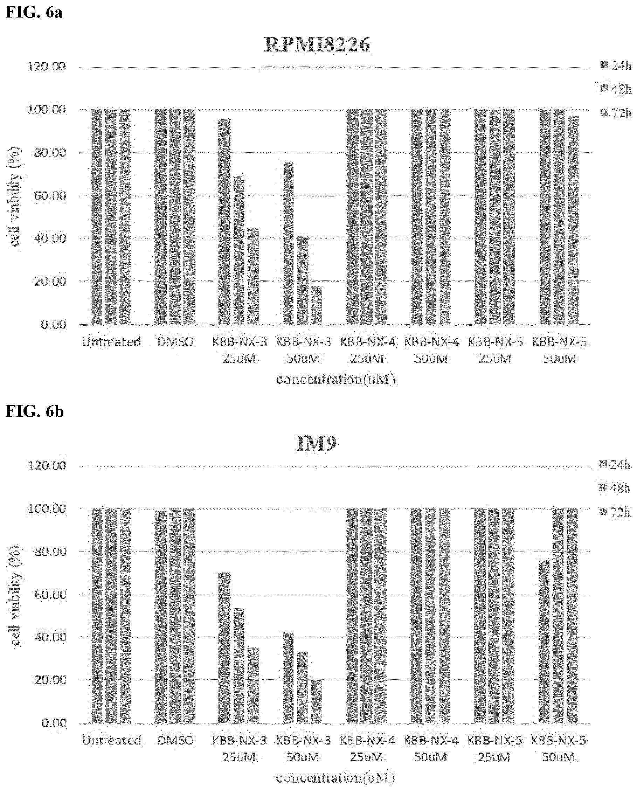

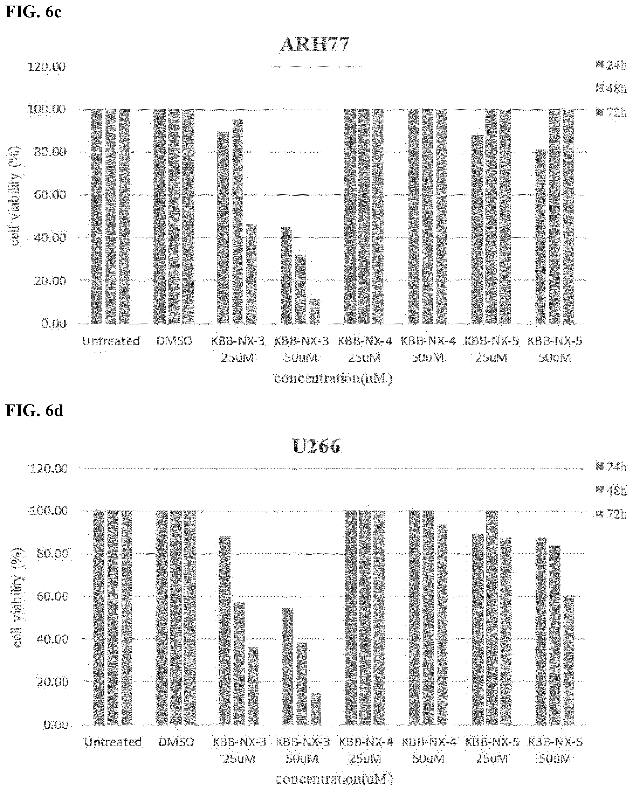

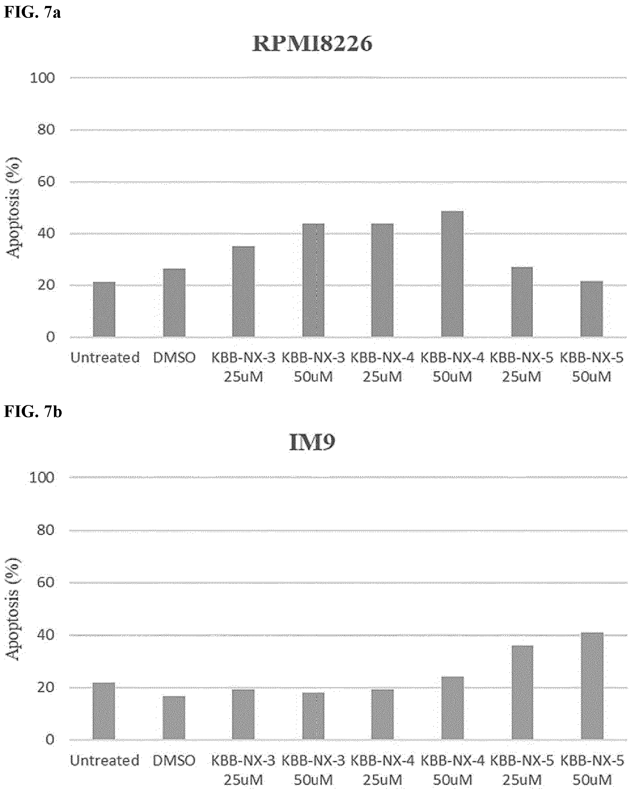

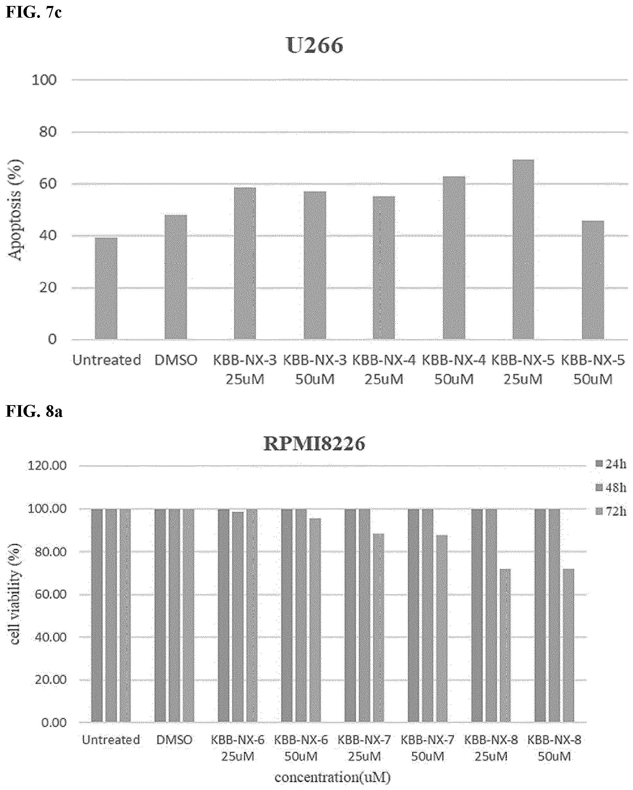

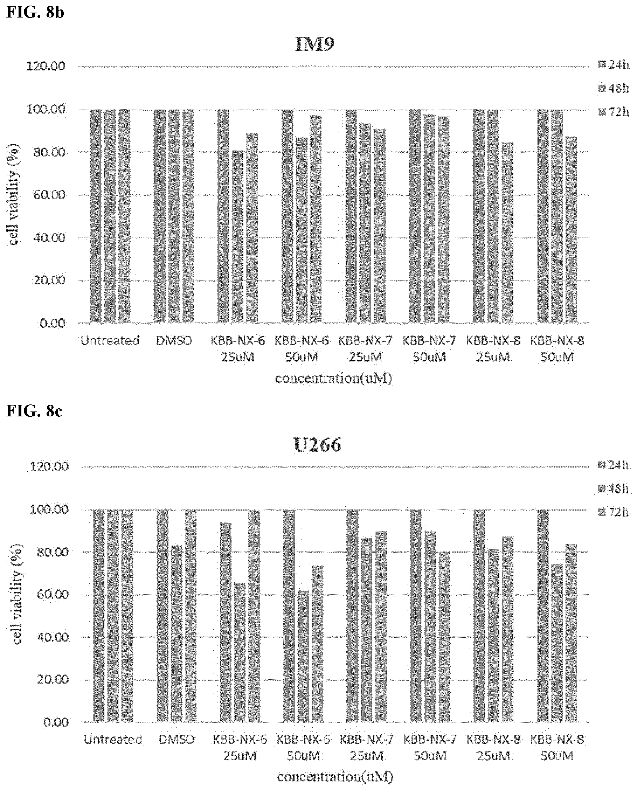

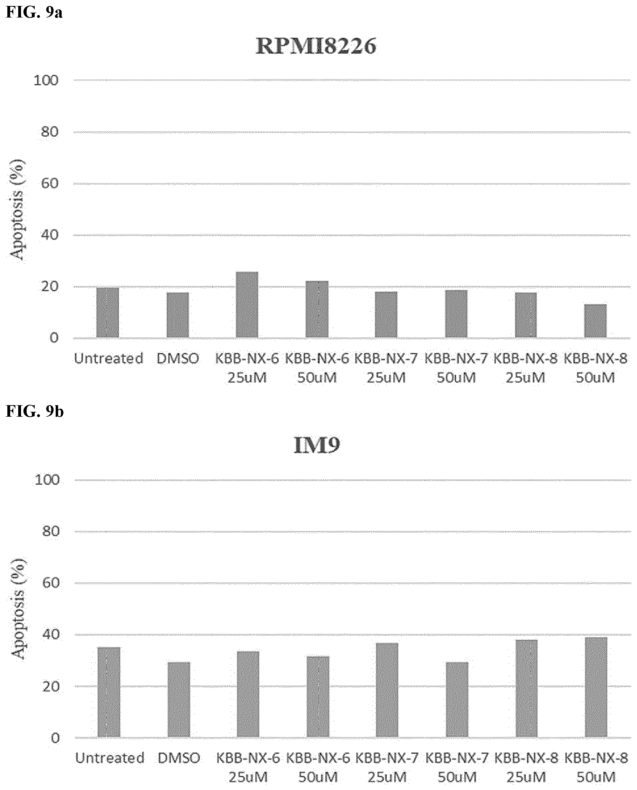

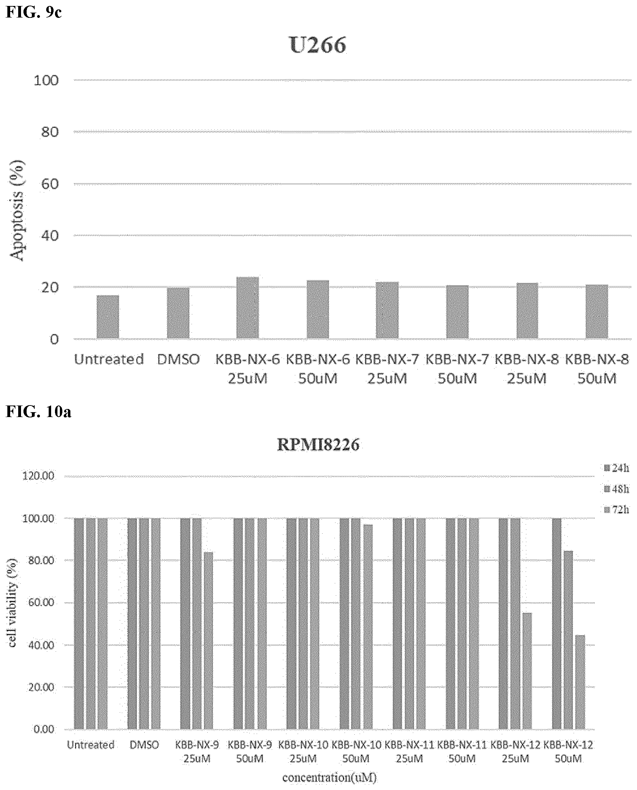

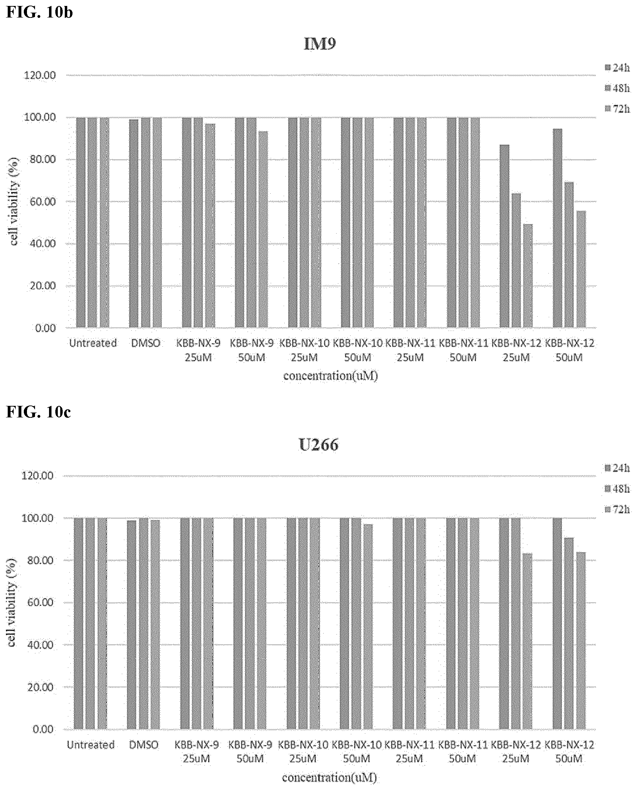

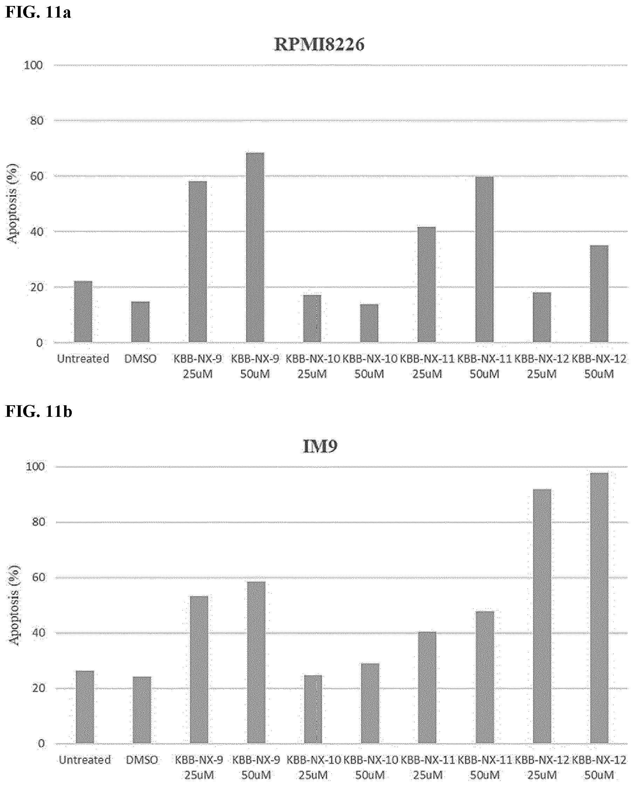

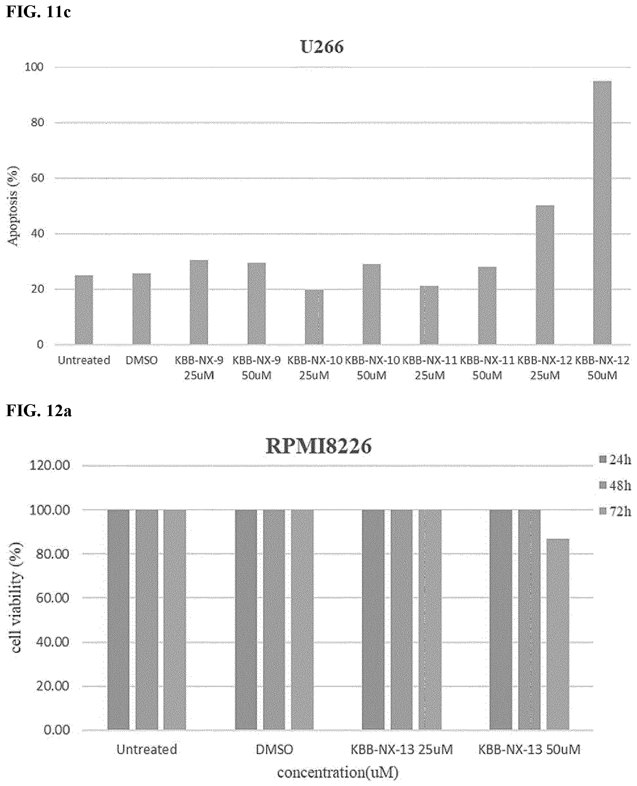

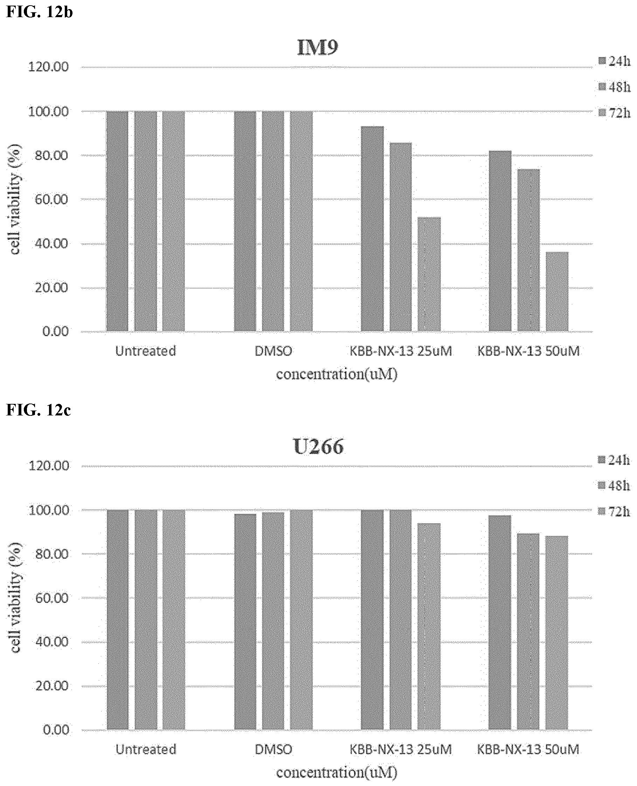

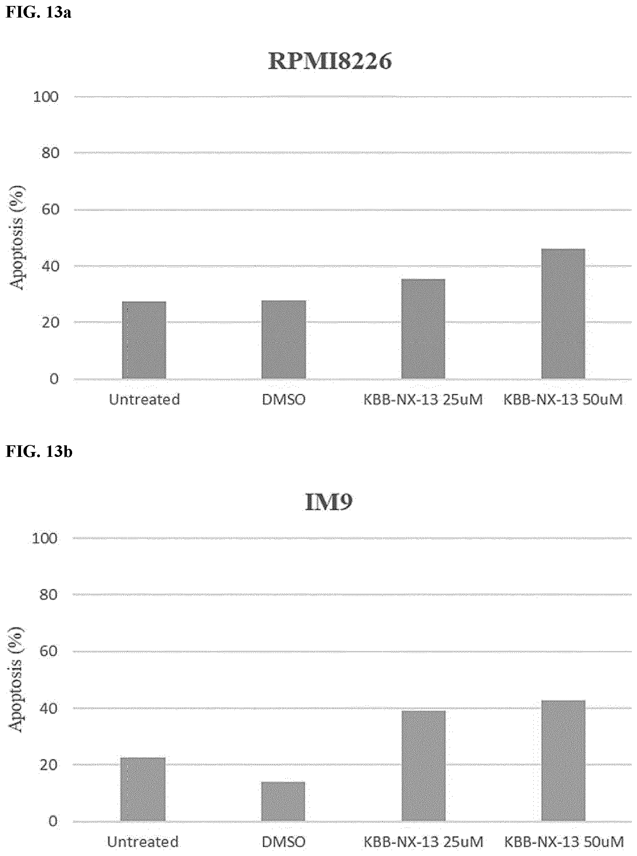

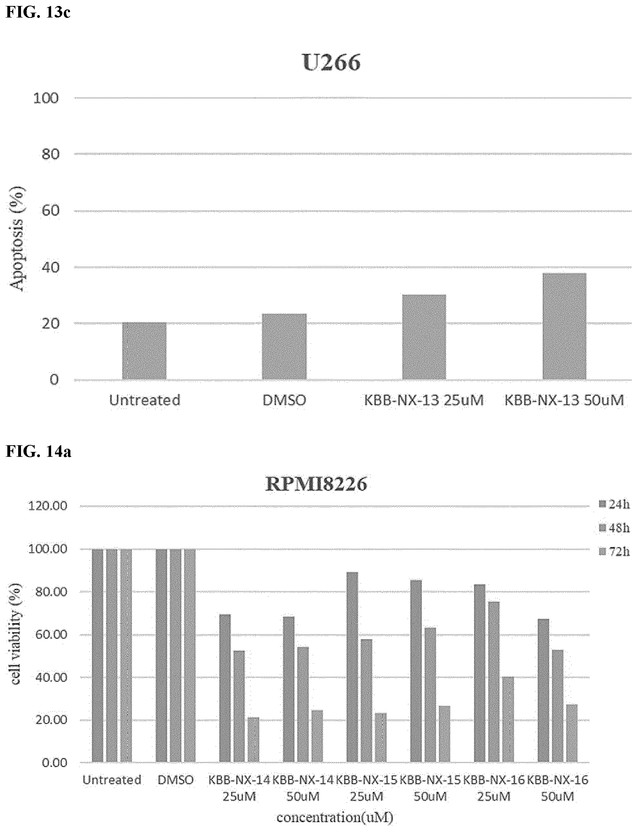

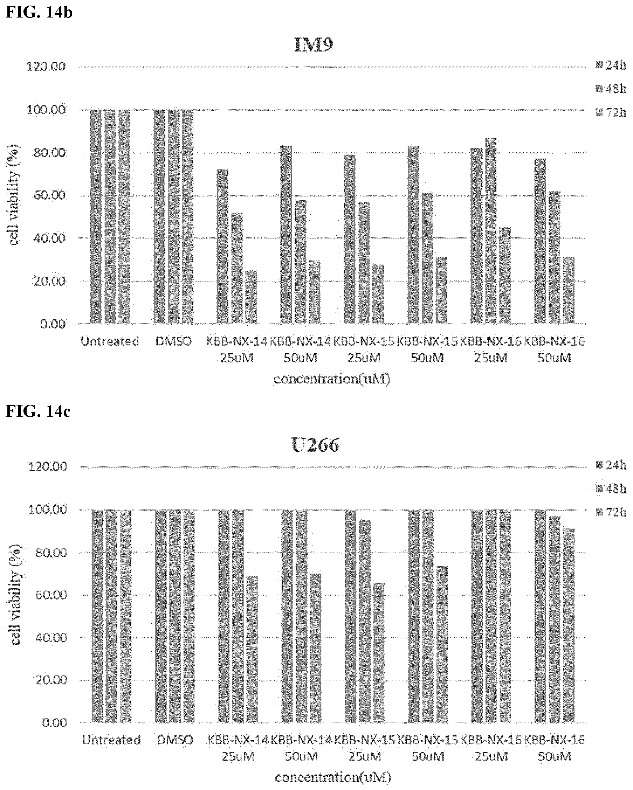

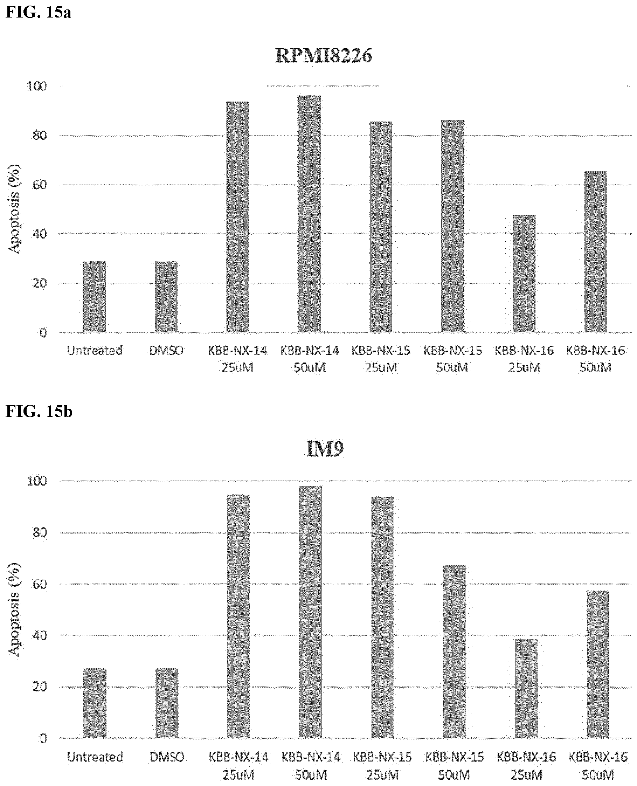

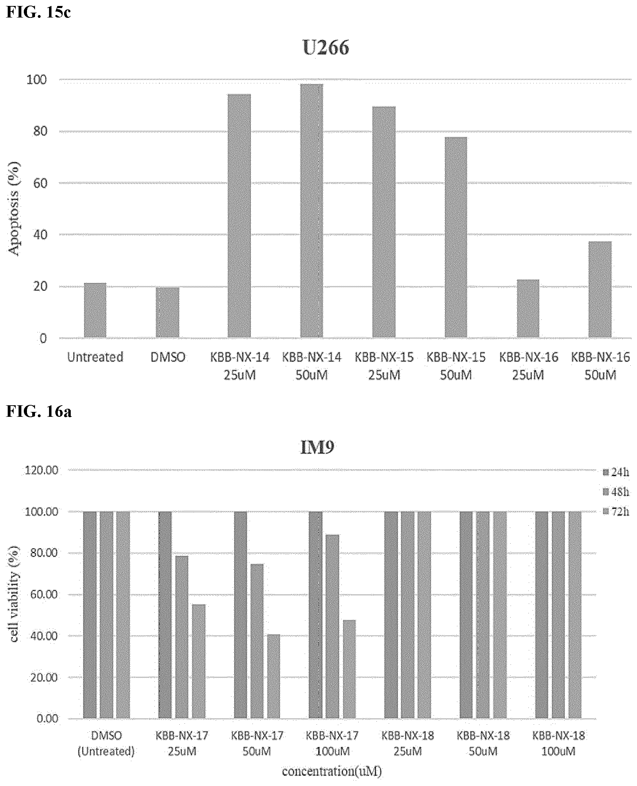

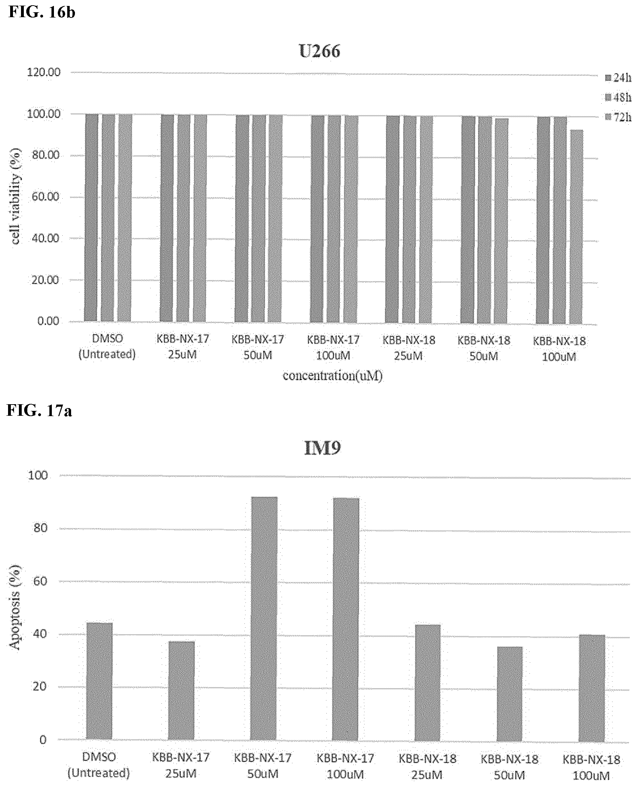

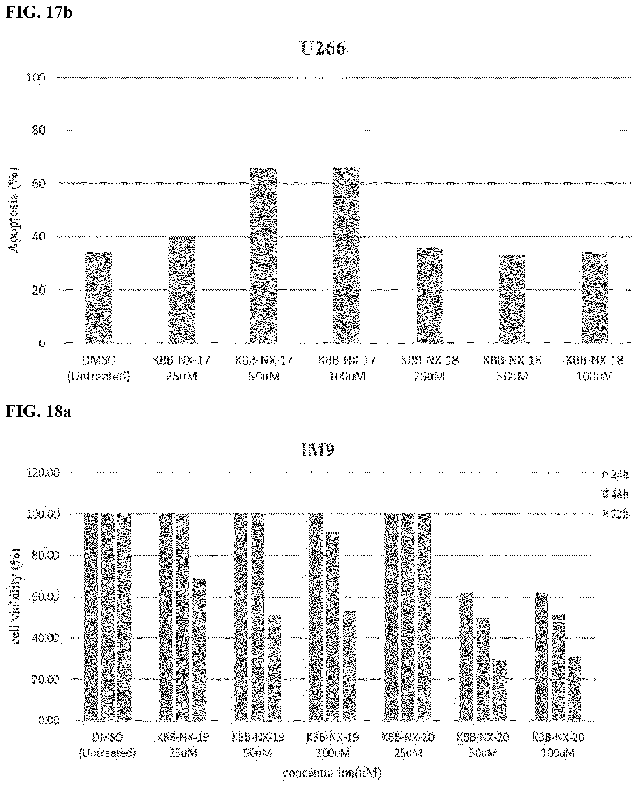

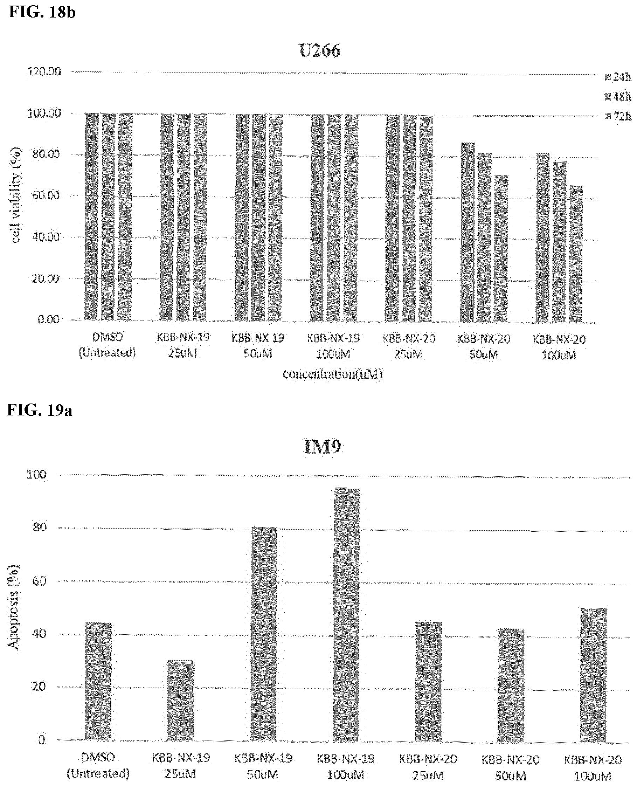

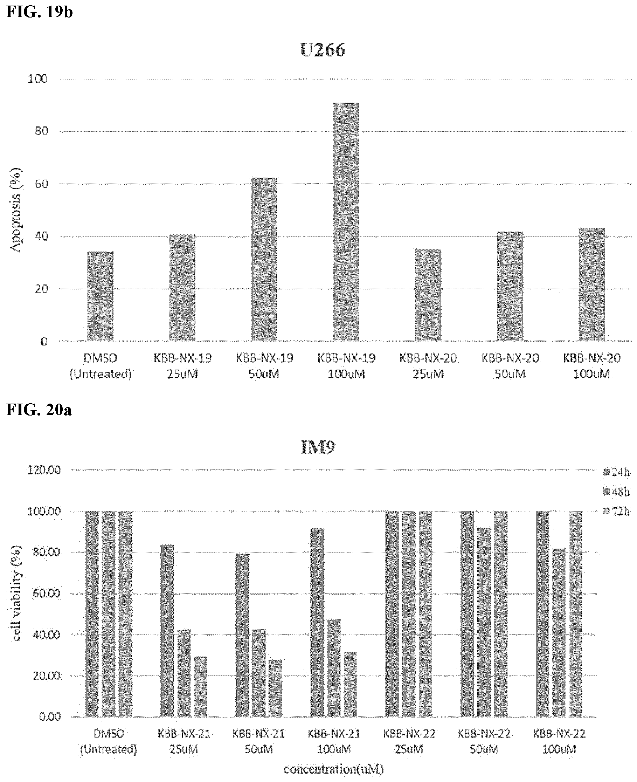

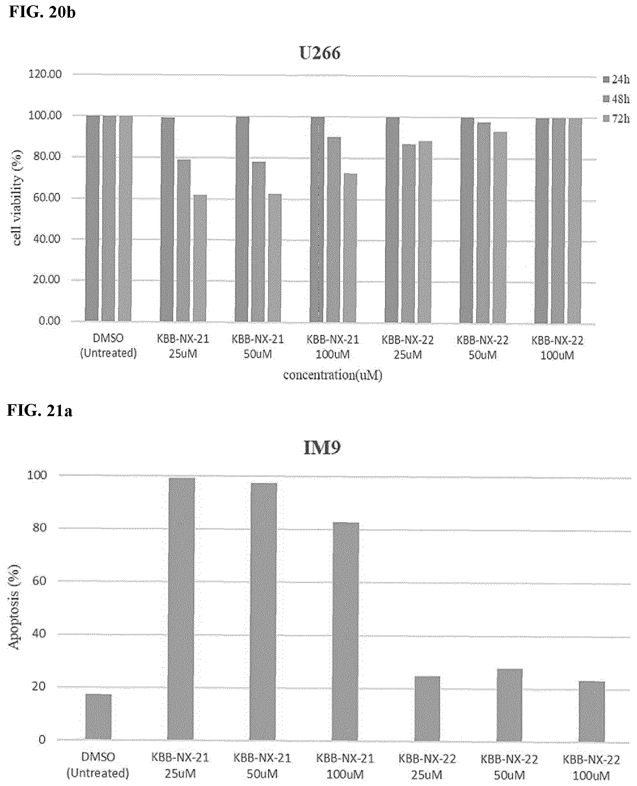

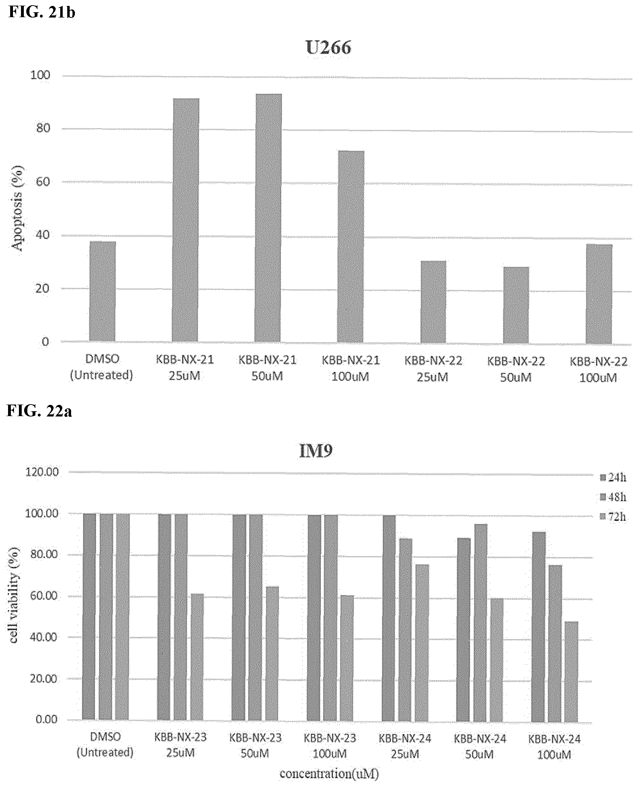

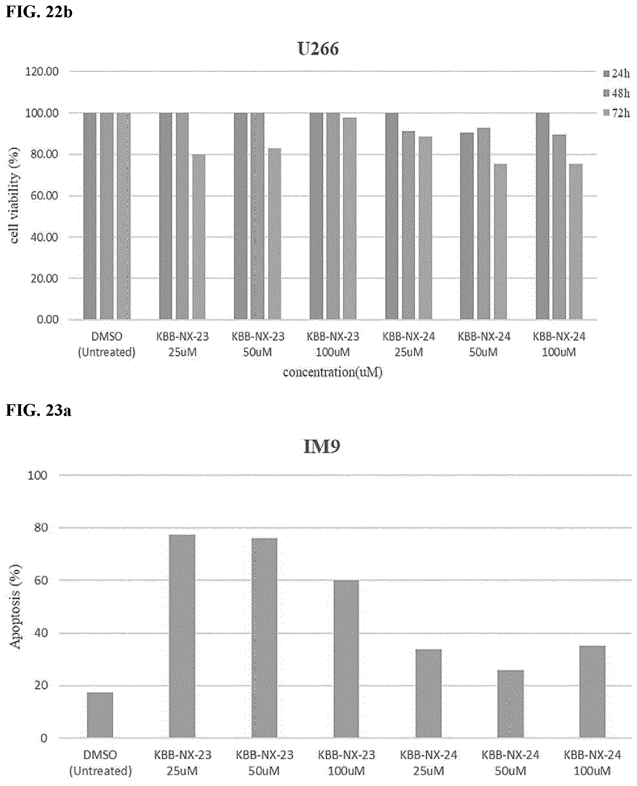

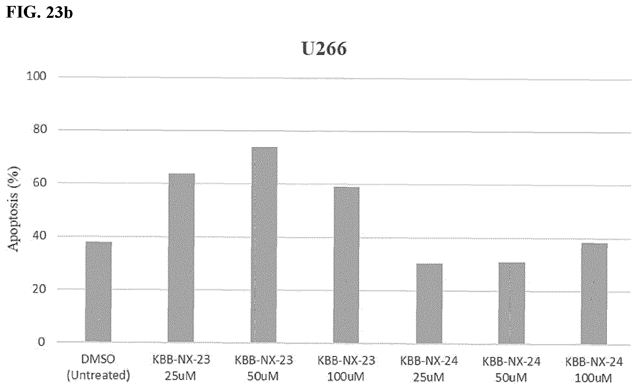

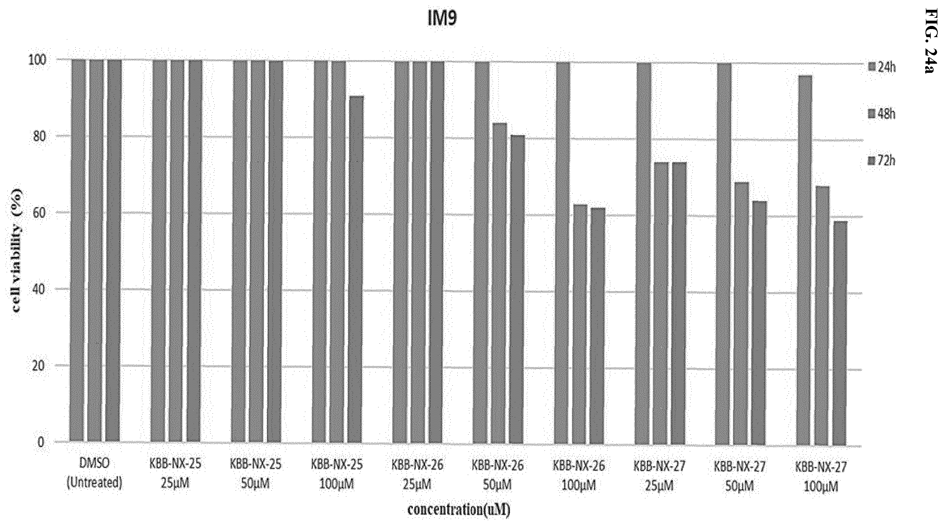

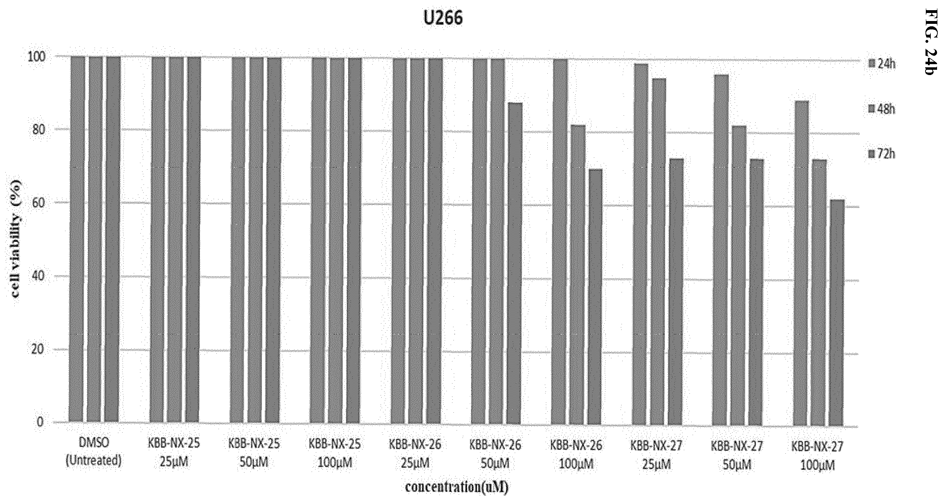

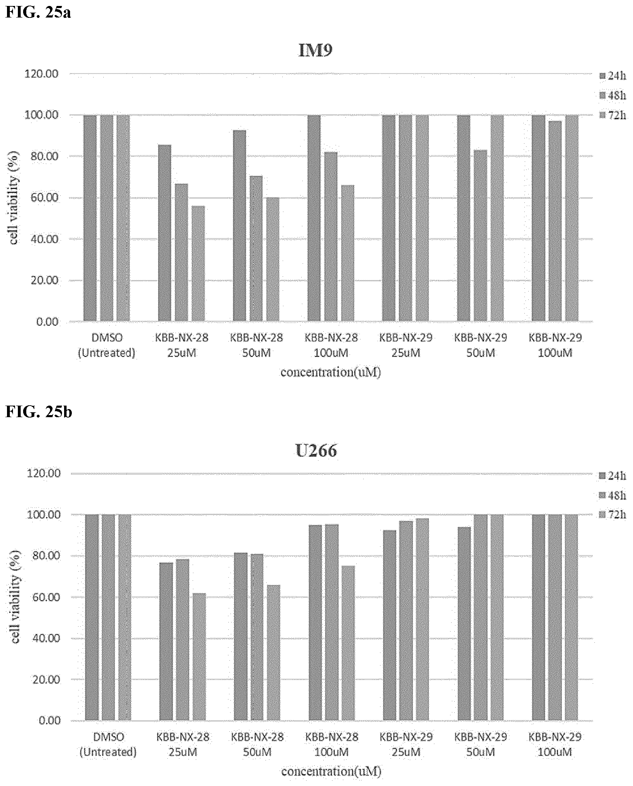

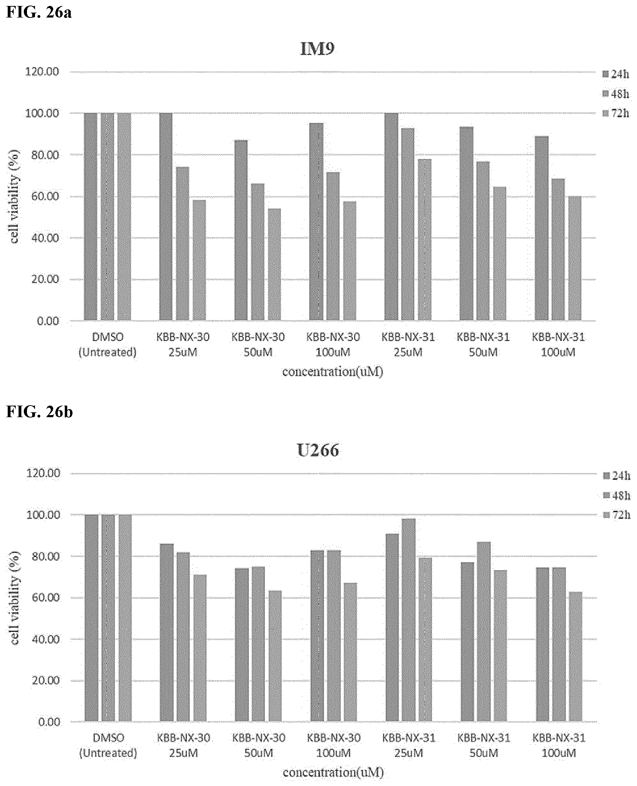

As shown in FIGS. 1 to 26, the compounds of the present invention were shown to have significant killing effects on the human multiple myeloma cell lines U266, IM9, RPMI 8226, and ARH-77 in diversified *in vitro* assays.

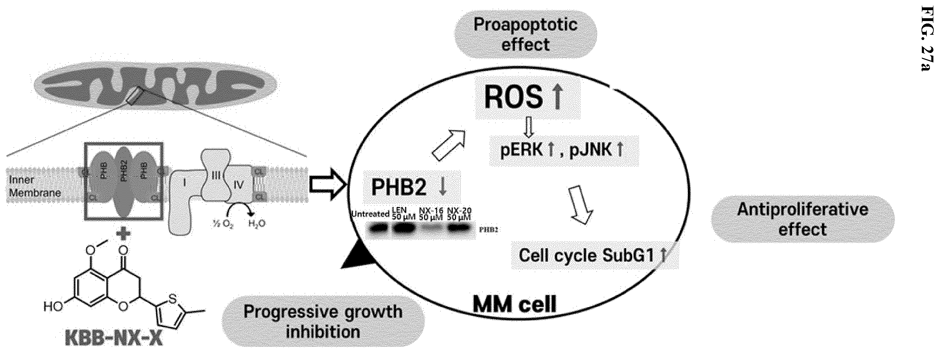

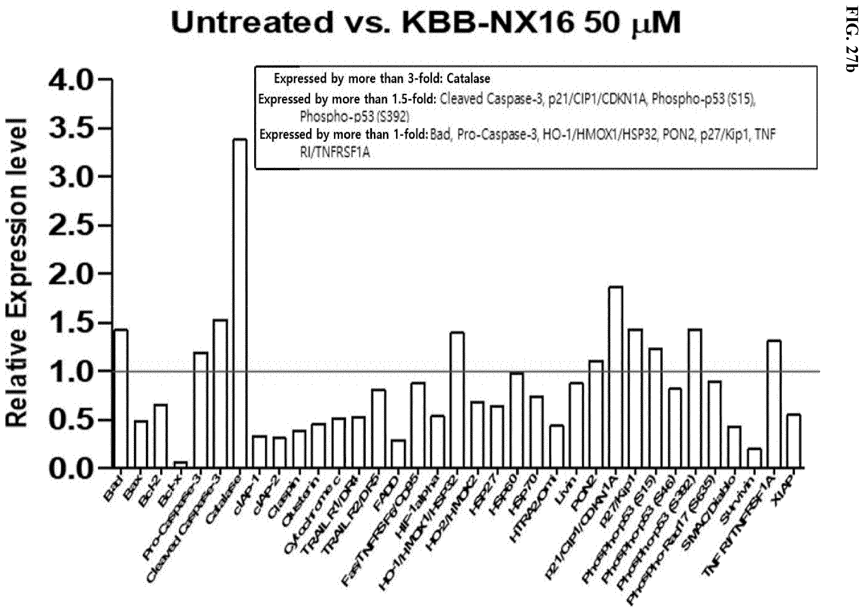

The mode of action by which the compounds of the present invention kill multiple myeloma cells is illustrated in FIG. 27. Briefly, KBB-NX compounds bind to the intracellular PHB2 protein (located primarily on the inner membrane of mitochondria) and neutralize PHB2 protein function, resulting in increased intracellular free radical generation and altered mitochondrial function. KBB-NX's antagonism of PHB2 increases intracellular ROS, resulting in cellular metabolic stress, which triggers a pathological compensatory mechanism inducing intracellular mitochondrial overproliferation, thereby generates excessive intracellular ROS and promotes ROS-mediated cell death. Increased intracellular ROS increases pERK and pJNK, which are key factors in the apoptosis pathway, promoting apoptosis (proapoptotic effect) and arresting the cell cycle in SubG1 phase (antiproliferative effect). Protein array analysis revealed that KBB-NX compound treatment induced PHB2 protein decrease - ROS increase mediated cell death (Figure 27b).

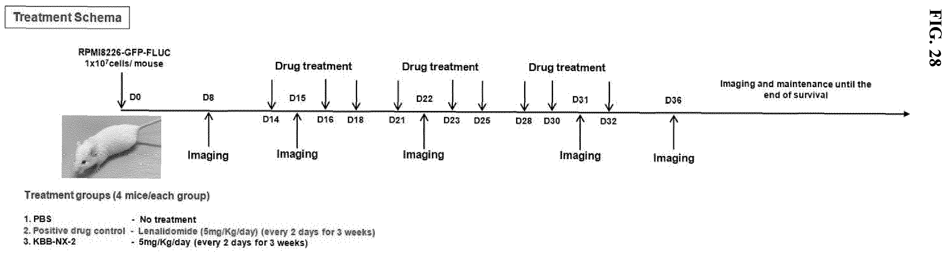

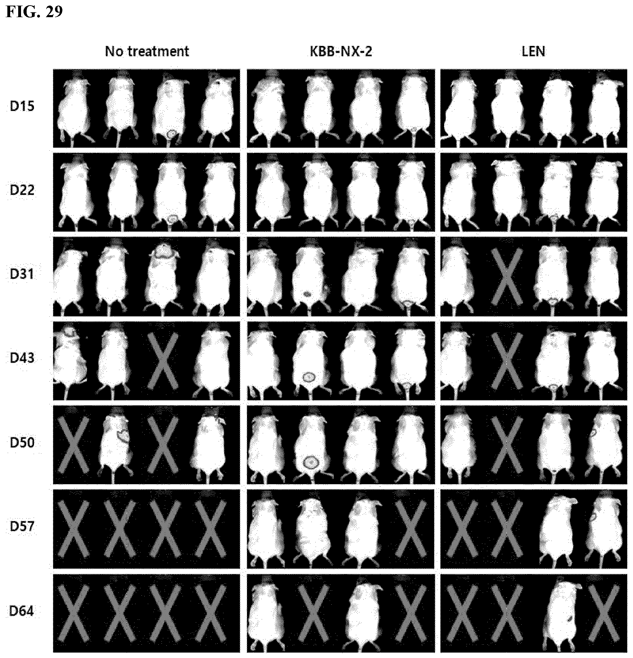

Meanwhile, a multiple myeloma cell line (RPMI8226) was transfected with a puromycin resistance gene and a luciferase-GFP gene using a lentiviral vector at the EF1a promoter site, and the dynamics of MM cells in mice were identified and monitored by an in vivo bioluminescence imaging assay (FIG. 28). The transgenic multiple myeloma cell line, which can be monitored *in vivo,* was injected 1 x 10⁷ cells/ ml through the tail vein of 6-11 week old combined immunodeficient NSG mice to establish a mouse model of multiple myeloma. As a result, all of the mice in the untreated group died on day 50 after transplantation, while the mice in the KBB-NX-2 and lenalidomide treatment groups survived until day 64, respectively, clearly confirming the effectiveness of KBB-NX compounds in treating multiple myeloma *in vitro* (FIG. 29).

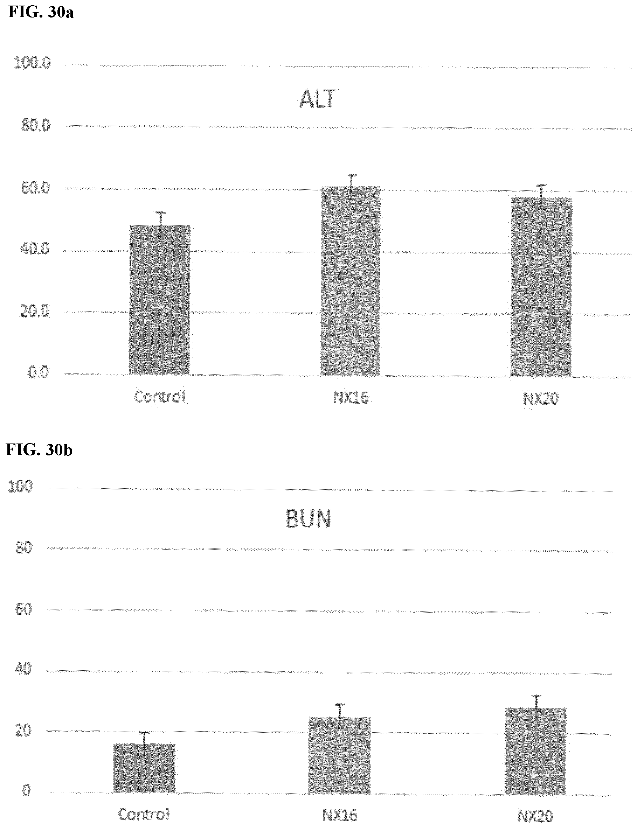

Furthermore, the compounds of the invention, including KBB-NX-16 and KBB-NX-20, did not exhibit significant hepatotoxicity, nephrotoxicity, and cardiotoxicity in the initial acute toxicity evaluation (FIG. 30).

Although the present invention has been described in detail with reference to the specific features, it will be apparent to those skilled in the art that this description is only of a preferred embodiment thereof, and does not limit the scope of the present invention. Thus, the substantial scope of the present invention will be defined by the appended claims and equivalents thereto.

## Claims

1. A compound represented by the following Formula 1 or a pharmaceutically acceptable salt thereof: 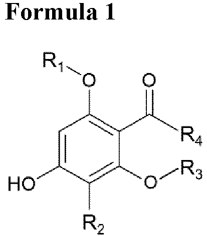 wherein R₁ is C₁-C₃ alkyl; R₂ is hydrogen or 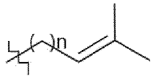 (where n is an integer of 0 to 2); R₃ is hydrogen and R₄ is 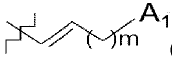 (where m is an integer of 0 to 2 and A₁ is C₆-C₁₀ aryl or 5 to 10-membered heteroaryl unsubstituted or substituted with hydroxy, halogen, C₁-C₃ alkyl, nitro, or 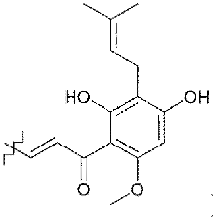 ), or R₃ and R₄ are bonded to each other to form a 6-membered heterocycloalkyl or heterocycloalkene substituted with C₆-C₁₀ aryl or 5 to 10-membered heteroaryl unsubstituted or substituted with hydroxy, halogen or C₁ -C₃ alkyl.

2. The compound or the pharmaceutically acceptable salt of claim 1, wherein the heteroaryl is selected from the group consisting of thiophene, furan, benzofuran and quinoline.

3. The compound or the pharmaceutically acceptable salt of claim 1, wherein n is 1 and m is 0.

4. The compound or the pharmaceutically acceptable salt of claim 1, wherein the heterocycloalkyl is oxanone and the heterocycloalkene is dihydropyranone.

5. The compound or the pharmaceutically acceptable salt of claim 1, wherein the compound represented by Formula 1 is a compound represented by the following Formula 2: 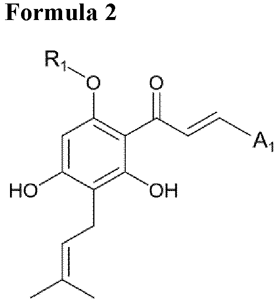 wherein R₁ and A₁ are as defined in Formula 1.

6. The compound or the pharmaceutically acceptable salt of claim 1, wherein the compound represented by Formula 1 is a compound represented by the following Formula 3: 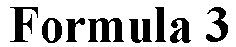 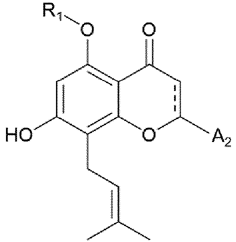 wherein R₁ is as defined in Formula 1, - - - is a single- or double bond, A₂ is C₆-C₁₀ aryl or 5 to 10-membered heteroaryl unsubstituted or substituted with hydroxy, halogen or C₁ -C₃ alkyl.

7. The compound or the pharmaceutically acceptable salt of claim 1, wherein the compound represented by Formula 1 is selected from the group consisting of compounds represented by the following Formula 4 to 33:

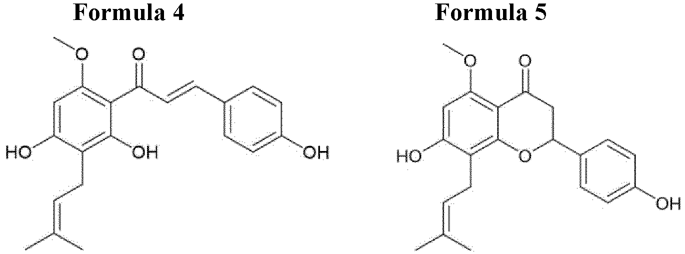

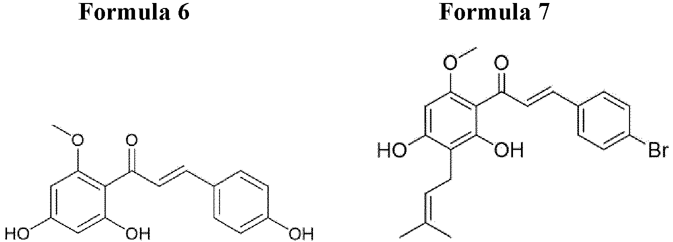

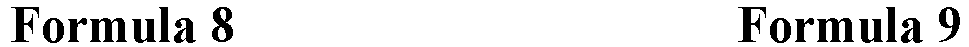

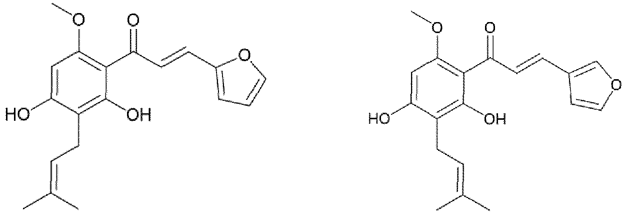

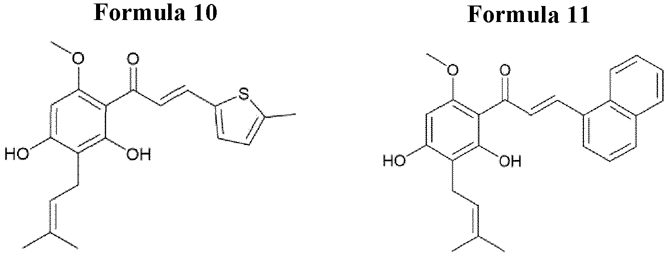

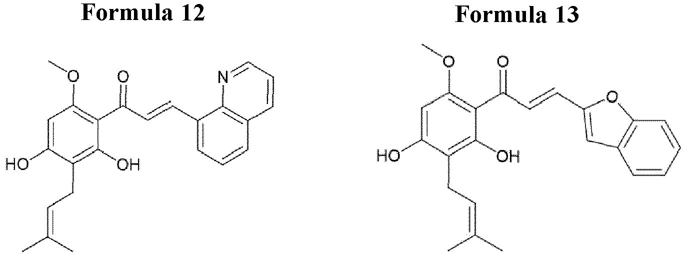

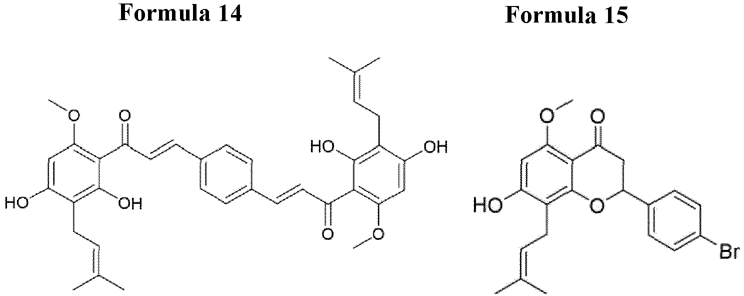

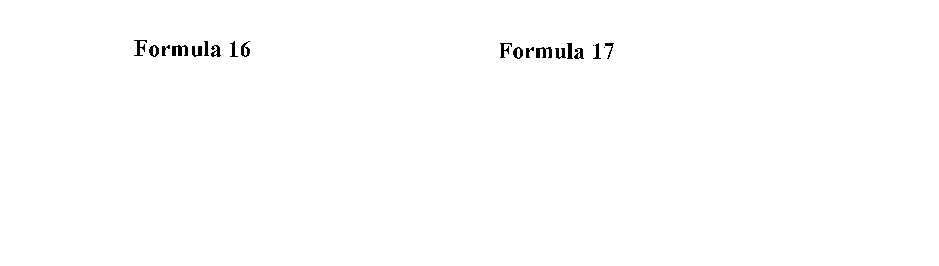

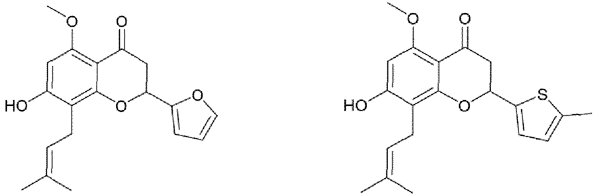

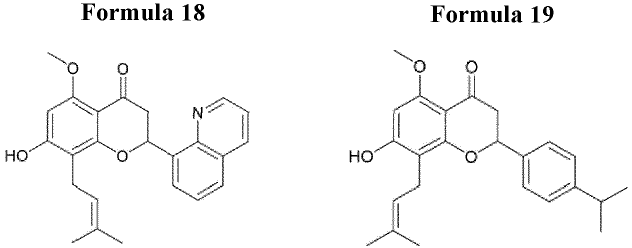

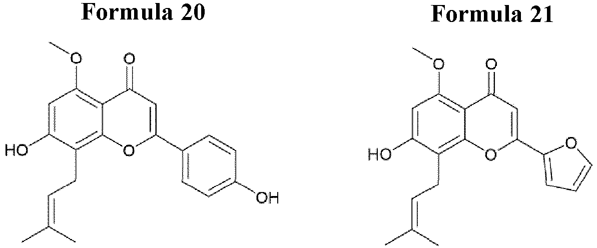

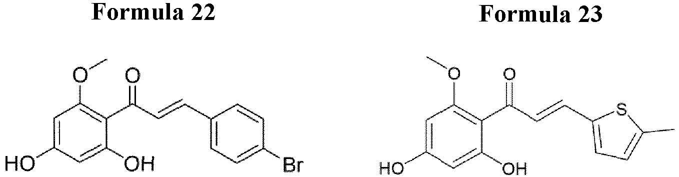

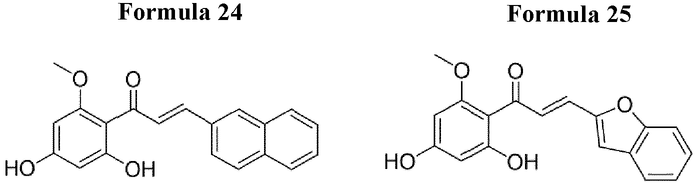

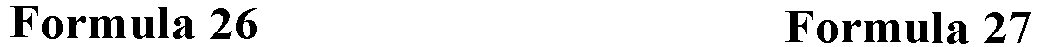

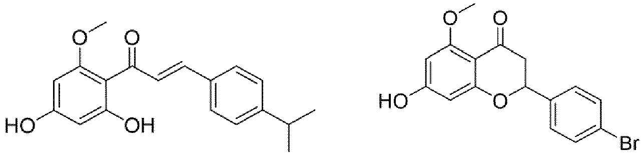

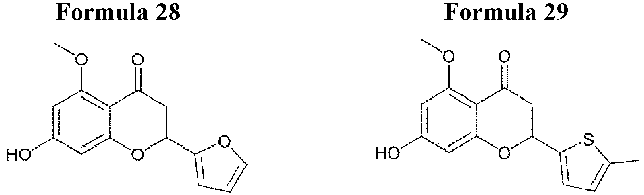

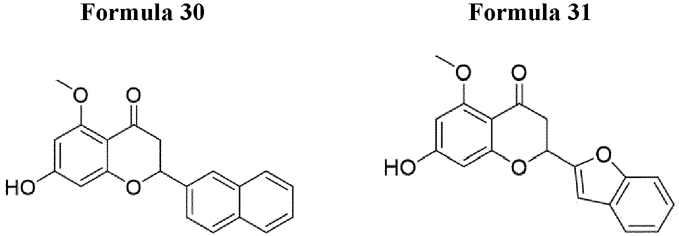

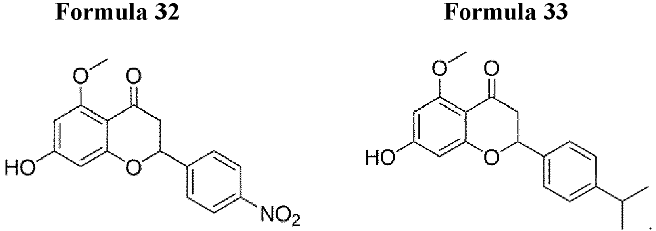

8. A composition for preventing or treating cancer comprising a compound or a pharmaceutically acceptable salt thereof of any one of claims 1 to 7, as an active ingredient.

9. The composition of claim 8, wherein the cancer is multiple myeloma.

10. The composition of claim 8, further comprising a compound represented by the following Formula 34 or a pharmaceutically acceptable salt thereof: 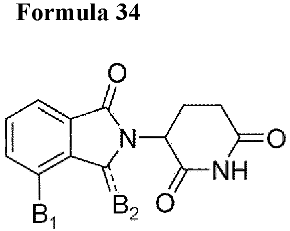 wherein B₁ is hydrogen or -NH₂, - - - is a single bond or double bond, and B₂ is hydrogen when - - - is a single bond, or B₂ is oxygen when - - - is a double bond.

11. An anti-cancer composition for co-administration with a compound represented by the following Formula 34 or a pharmaceutically acceptable salt thereof, comprising a compound or a pharmaceutically acceptable salt thereof of any one of claims 1 to 7, as an active ingredient; 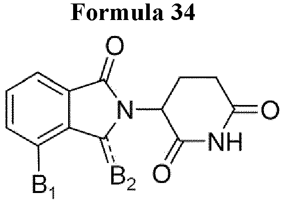 wherein B₁ is hydrogen or -NH₂, - - - is a single bond or double bond, and B₂ is hydrogen when - - - is a single bond, or B₂ is oxygen when - - - is a double bond.

12. The composition of claim 11, wherein the composition is a composition for preventing or treating multiple myeloma.
